# EUROPEAN PATENT SPECIFICATION

(11) **EP 1 770 129 B1**
(45) Date of publication and mention of the grant of the patent: **25.07.2012**
(21) Application number: 06025328.3
(22) Date of filing: 28.09.2001
(51) Int. Cl.: C09B 23/02, G01N 33/58, G01N 33/533, C07D 209/18, C12Q 1/68

(54) **Modified carbocyanine dyes and their conjugates**
Modifizierte Carbocyaninfarbstoffe und deren Konjugate
Colorants carbocyanines modifies et leurs conjugues

(30) Priority: 29.09.2000 US 236637 P; 16.03.2001 US 276870 P
(43) Date of publication of application: 04.04.2007
(62) Divisional of application: 01975541.2
(73) Proprietor: Life Technologies Corporation, Carlsbad, CA 92008 (US)
(72) Inventor: Leung, Wai-Yee, San Ramon, CA 94583 (US); Cheung, Ching-Ying, San Ramon, CA 94583 (US); Yue, Stephen, Eugene, OR 97405 (US)
(74) Representative: HOFFMANN EITLE

(56) References cited:
- WO-A-96/17628
- WO-A-98/48838
- DE-A- 19 937 024
- JP-A- 5 313 304
- US-A- 5 268 486
- US-A- 5 627 027
- MUJUMDAR R B ET AL: "CYANINE DYE LABELING REAGENTS: SULFOINDOCYANINE SUCCINIMIDYL ESTERS" BIOCONJUGATE CHEMISTRY, AMERICAN CHEMICAL SOCIETY, WASHINGTON, US, vol. 4, no. 2, 1 March 1993 (1993-03-01), pages 105-111, XP000654181 ISSN: 1043-1802
- H.J. GRUBER ET AL.: "Anomalous Fluorescence Enhancement of Cy3 and Cy3.5..." BIOCONJUGATE CHEMISTRY., vol. 11, 2000, pages 696-704, XP002191648 AMERICAN CHEMICAL SOCIETY, WASHINGTON., US ISSN: 1043-1802

## Description

### FIELD OF THE INVENTION

The invention relates to colored and fluorescent chemicals, including reactive dyes and dye-conjugates; and to their uses.

### BACKGROUND OF THE INVENTION

Fluorescent compounds are covalently or noncovalently attached to other materials to impart color and fluorescence. Brightly fluorescent dyes permit detection or location of the attached materials with great sensitivity. Certain carbocyanine dyes have demonstrated utility as labeling reagents for a variety of biological applications, e.g. U.S. Patents 4,981,977 to Southwick, et al. (1991); 5,268,486 to Waggoner et al. (1993); 5,569,587 to Waggoner (1996); 5,569,766 to Waggoner et al. 91996); 5,486,816 to Waggoner et al. (1998); 5,627,027 to Waggoner (1997); 5,808,044 to Brush, et al. (1998); 5,877,310 to Reddington, et al. (1999); 6,002,003 to Shen, et al. 91999); 6,004,536 to Leung et al. 91999); 6,008,373 to Waggoner, et al. (1999); 6,043,025 to Minden, et al. (2000); 6,127,134 to Minden, et al. (2000); 6,130,094 to Waggoner, et al. (2000); 6,133,445 to Waggoner, et al. (2000); also WO 97/40104, WO 99/51702, WO 01/21624, and EP 1065 250A1; and TETRAHEDRON LETTERS 41, 9185-88 (2000) . Nevertheless, many carbocyanine dyes are known to share certain disadvantages, e.g. severe quenching of the fluorescence of carbocyanine dyes in biopolymer conjugates, e.g. quenching of Cy5 and Cy7 dye variants on conjugates, as discussed by Gruber et al, BIOCONJUGATE CHEM. 11,696 (2000), and in EP 1 065250 A1, 0004. In addition, certain desired sulfoalkyl derivatives of the reactive carbocyanine dyes are difficult to prepare, as indicated for Cy3 andCy5 variants by Waggoner and colleagues in BIOCONJUGATE CHEM. 4, 105, 109 col.2 (1993). Cyanine dyes also have a very strong tendency to self-associate (i.e. stack), which can significantly reduce the fluorescence quantum yields, as described in the extensive review by Mishra, et al., CHEM. REV. 100, 1973 (2000).

WO96/17628 discloses an *in vivo* diagnostic method based on near infrared radiation (near I.R. radiation) uses water-soluble dyes and their biomolecular adducts having certain photophysical and pharmacochemical properties as contrasting agents for fluorescence and transillumination diagnosis in the near infrared range. Also disclosed are new dyes and pharmaceuticals containing the same.

JP05 313 304 discloses a silver halide photographic infrared sensitive material stable in storage and free from residual dye stains after development processing by incorporating a specified dye. The dye is added to the reverse side of a support or between the support and an emulsion layer, and it is effective as a filter dye and it is used, preferably together with a binder.

WO 98/48838 discloses a physiologically tolerable light imaging contrast agent compound having a molecular weight in the range 500 to 500000 and containing at least two.chromophores having delocalized electron systems as well as at least one polyalkylene oxide (PAO) moiety having a molecular weight in the range 60 to 100000.

Modification of an indolium ring of the carbocyanine dye to permit a reactive group or conjugated substance at the number 3 position unexpectedly mitigates these problems and results in dye-conjugates that are uniformly and substantially more fluorescent on proteins, nucleic acids and other biopolymers, than conjugates labeled with structurally similar carbocyanine dyes bound through the nitrogen atom at the number 1 position. In addition to having more intense fluorescence emission than structurally similar dyes at virtually identical wavelengths, and decreased artifacts in their absorption spectra upon conjugation to biopolymers, certain embodiments of the invention also have greater photostability and higher absorbance (extinction coefficients) at the wavelength(s) of peak absorbance than such structurally similar dyes. These improvements result in significantly greater sensitivity in assays that use these dyes and their conjugates, while utilizing available filters and instrumentation already commercially available for use with similar dyes.

Furthermore, the dyes of the invention typically exhibit absorbance maxima between about 530 nm and about 800 nm, so dyes can be selected to match the principal emission lines of the mercury arc lamp (546 nm), frequency-doubled Nd-Yag laser (532 nm), Kr-ion laser (568 nm and 647 nm), HeNe laser (543 nm, 594 nm, and 633 nm) or long-wavelength laser diodes (especially 635 nm and longer), The azacarbocyanine dyes of the invention exhibit a bathochromic spectral shift (a shift to longer wavelength) of approximately 20 to 50 nm relative to otherwise structurally similar carbocyanine dyes known in the art. Some dyes of the invention exhibit very long wavelength excitation (at least 640 nm, but some greater than about 780 nm) and emission bands (at least 665 nm, and some greater than about 750 nm), so they are particularly useful for samples that are transparent to infrared wavelengths.

### DESCRIPTION OF DRAWINGS

Figure 1. Direct comparison of absorbance properties of Compound 30 (Cy5-type linkage, open circles) with Compound 24 (of the invention, closed circles) when conjugated to GAR at DOS's of approximately 2.8,4.3, and 5.5 (600 nm absorbance bands increasing as a function of increasing DOS, 650 nm absorbance bands normalized to 1.0) (see Example 47).
Figure 2. Comparison of the fluorescence of goat anti-rabbit IgG (GAR) conjugates of Compound 13 Reference dye (solid line) and those of the spectrally similar CY3 dye (dashed line) (see Example 49).
Figure 3. photostability comparison of Compound 9 Reference dye (solid circles) with Cy5 (open circles), (40X objective, pH = 7.5 solution) (see Example 50).
Figure 4. Comparison of the change in absorbance of Compound 9 (closed circles) and Cy5 (open circles) upon DNA incorporation using ULS methodology (see Example 58).
Figure 5. Comparison of the absorption and fluorescence properties of identical concentrations of Compound 9 (solid line) and Cy5 (dashed line) labeled cDNA (see Example 60).

### SUMMARY OF TEE INVENTION AND DESCRIPTION OF PREFERRED EMBODIMENTS

The present invention describes modified carbocyanine dyes and their conjugates. The dyes and dye conjugates are used to locate or detect the interaction or presence of analytes or ligands in a sample. Kits incorporating such dyes or dye conjugates facilitate their use in such methods.

### Dyes

The carbocyanine dyes of the invention are defined by the appended claims.

By "sulfo" is meant sulfonic acid, or salt of sulfonic acid (sulfonate). Similarly, by "carboxy" is meant carboxylic acid or salt of carboxylic acid. "Phosphate", as used herein, is an ester of phosphoric acid, and includes salts of phosphate. "Phosphonate", as used herein, means phosphonic acid and includes salts of phosphonate. As used herein, unless otherwise specified, the alkyl portions of substituents such as alkyl, alkoxy, arylalkyl, alylamino, dialkylamino, trialkylammonium, or perfluoroalkyl are optionally saturated, unsaturated, linear or branched, and all alkyl, alkoxy, alkylamino, and dialkylamino substituents are optionally themselves further substituted by carboxy, sulfo, amino, or hydroxy.

Preferably, R⁴, R¹³ and R¹⁴ are independently alkyl having 1-6 carbons, optionally substituted one or more times by fluorine, chlorine, bromine, iodine, hydroxy, carboxy, sulfo, or amino; more preferably R⁴, R¹³ and R¹⁴ are methyl or ethyl. In one aspect of the invention, R⁴, R¹³ and R¹⁴ are methyl.

R² and R¹² are the same and are methyl or ethyl.

The length of the polymethine bridge between the heterocyclic ring systems affects the dye's absorption and emission properties. n=1, 2, or 3. Where n is >3, the dyes have spectra even further shifted into the infrared region. In a preferred aspect of the azacarbocyanine dyes, n is 2.

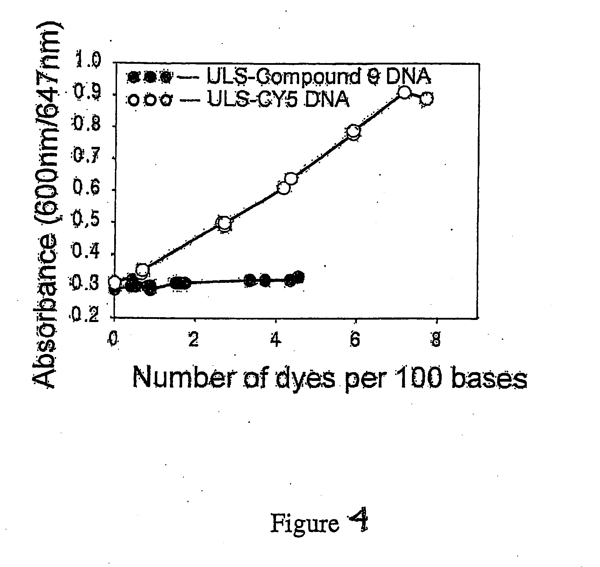

The dye is sulfonated at both R⁷ or R¹⁷. Generally, commercially available reactive carbocyanine dyes are sulfonated up to three times (at positions corresponding to R⁷ and R¹⁷, and as sulfoalkyl at one of R² and R¹²), leaving one of R² and R¹² for the location of the reactive group. In contrast, by attaching the reactive group (or conjugated substance) at R³, certain carbocyanine dyes of the invention may be sulfonated at least four times (at R⁷, at R¹⁷, and as sulfoakyl at R² and R¹²). This extra sulfonation, as well as the change in attachment site, results in reactive dyes and dye conjugates that are brighter, more soluble in aqueous solutions, and more resistant to the fluorescence quenching that results from dye-dye stacking interactions. However, sulfonation by four or more sulfonic acids is not required for the dyes of the invention to have spectral properties that are superior to those of structurally similar dyes that are not linked through the 3 position of the indolium ring (Figure 4).

In addition, the dyes of the invention are substituted by one chemically reactive group (-L-Rₓ₎ or conjugated substance (-L-S_{c}), as described below.

Many embodiments of the compounds of the invention possess an overall electronic charge. It is to be understood that when such electronic charges are shown to be present, they are balanced by the presence of an appropriate counterion, which may or may not be explicitly identified. A biologically compatible counterion, which is preferred for some applications, is not toxic in biological applications, and does not have a substantially deleterious effect on biomolecules. Where the compound of the invention is positively charged, the counterion is typically selected from, but not limited to, chloride, bromide, iodide, sulfate, alkanesulfonate, arylsulfonate, phosphate, perchlorate, tetrafluoroborate, tetraarylboride, nitrate and anions of aromatic or aliphatic carboxylic acids. Where the compound of the invention is negatively charged, the counterion is typically selected from, but not limited to, alkali metal ions, alkaline earth metal ions, transition metal ions, ammonium or substituted ammonium or pyridinium ions. Preferably, any necessary counterion is biologically compatible, is not toxic as used, and does not have a substantially deleterious effect on biomolecules. Counterions are readily changed by methods well known in the art, such as ion-exchange chromatography, or selective precipitation.

It is to be understood that the dyes of the invention have been drawn in one or another particular electronic resonance structure. Every aspect of the instant invention applies equally to dyes that are formally drawn with other permitted resonance structures, as the electronic charge on the subject dyes are delocalized throughout the dye itself.

**Table 1. Spectral properties of selected dyes**

| Cpd No. | Excitation (nm, MeOH) | Emission (nm, MeOH) | Quantum Yield† (MeOH) |
|---|---|---|---|
| 66* | 686 | 706 | 0.35 |
| 68* | 664 | 696 | 0.4 |
| 72* | 663 | 694 | 0.42 |
| 73* | 664 | 697 | 0.48 |

| | | | |
|---|---|---|---|
| † Relative to nile blue in ethanol and CY5 dye in methanol | | | |

| | | | |
|---|---|---|---|
| * (Reference examples) | | | |

### Conjugates of Reactive Dyes

In one embodiment of the invention, the dye contains one group -L-Rₓ, where Rₓ is the reactive group that is attached to the dye by a covalent linkage L. In certain embodiments, the covalent linkage attaching the dye to Rₓ contains multiple intervening atoms that serve as a spacer. The dyes with a reactive group (Rₓ) label a wide variety of organic or inorganic substances that contain or are modified to contain functional groups with suitable reactivity; resulting in chemical attachment of the conjugated substance (S_{c}), represented by -L-S_{c}. As used herein, "reactive group" means moiety on the compound that is capable of chemically reacting with a functional group on a different compound to form a covalent linkage. Typically the reactive group is an electrophile or nucleophile that can form a covalent linkage through exposure to the corresponding functional group that is a nucleophile or electroplate, respectively. Alternatively, the reactive group is a photoactivatable group, and becomes chemically reactive only after illumination with light of an appropriate wavelength. Typically, the conjugation reaction between the reactive dye and the substance to be conjugated results in one or more atoms of the reactive group Rₓ to be incorporated into a new linkage L attaching the dye to the conjugated substance Sₐ. Selected examples of reactive groups and linkages are shown in Table 2, where the reaction of an electrophilic group and a nucleophilic group yields a covalent linkage.

**Table 2: Examples of some routes to useful covalent linkages**

| Electrophilic Group | Nucleophilic Group | Resulting Covalent Linkage |
|---|---|---|
| activated esters* | amines/anilines | carboxamides |
| acrylamides | thiols | thioethers |
| acyl azides** | ammes/amtines | carboxamides |
| acyl halides | amines/anilines | carboxamides |
| acyl halides | alcohols/phenols | esters |
| acyl nitriles | alcohols/phenols | esters |
| acyl nitriles | amines/anilines | carboxamides |
| aldehydes | amines/anilines | imines |
| aldehydes or ketones | hydrazines | hydrazones |
| aldehydes or ketones | hydroxylamines | oximes |
| alkyl halides | amines/anilines | alkyl amines |
| alkyl halides | carboxylic acids | esters |
| alkyl halides | thiols | thioethers |
| alkyl halides | alcohols/phenols | ethers |
| alkyl sulfonates | thiols | thioethers |
| alkyl sulfonates | carboxylic acids | esters |
| alkyl sulfonates | alcohols/phenols | ethers |
| anhydrides | alcohols/phenols | esters |
| anhydrides | amines/anilines | carboxamides |
| aryl halides | thiols | thiophenols |
| aryl halides | amines | aryl amines |
| aziridines | thiols | thioethers |
| boronates | glycols | boronate esters |
| carbodiimides | carboxylic acids | N-acylureas or anhydrides |
| diazoalkanes | carboxylic acids | esters |
| epoxides | thiols | thioethers |
| haloacetamides | thiols | thioethers |
| haloplatinate | amino | platinum complex |
| haloplatinate | heterocycle | platinum complex |
| haloplatinate | thiol | platinum complex |
| halotriazines | amines/anilines | aminotriazines |
| halotriazines | alcohols/phenols | triazinyl ethers |
| imido esters | amines/anilines | amidines |
| isocyanates | ammes/anilines | ureas |
| isocyanates | alcohols/phenols | urethanes |
| isothiocyanates | amines/anilines | thioureas |
| maleimides | thiols | thioethers |
| phosphoramidites | alcohols | phosphite esters |
| silyl halides | alcohols | silyl ethers |
| sulfonate esters | amines/anilines | alkyl amines |
| sulfonate esters | thiols | thioethers |
| sulfonate esters | carboxylic acids | esters |
| sulfonate esters | alcohols | ethers |
| sulfonyl halides | amines/anilines | sulfonamides |
| sulfonyl halides | phenols/alcohols | sulfonate esters |

| | | |
|---|---|---|
| * Activated esters, as understood in the art, generally have the formula -COΩ, where Ω is a good leaving group (e.g. succinimidyloxy (-OC₄H₄O₂) sulfosuccinimidyloxy (-OC₄H₃O₂-SO₃H), -1-oxybenzotriazolyl (-OC₆H₄N₈); or an aryloxy group or aryloxy substituted one or more times by electron withdrawing substituents such as nitro, fluoro, chloro, cyano, or trifluoromethyl, or combinations thereof, used to form activated aryl esters; or a carboxylic acid activated by a carbodiimide to form an anhydride or mixed anhydride -OCOR^{a} or -OCNR^{a}NHR^{b}, where R^{a} and R^{b}, which may be the same or different, are C₁-C₆ alkyl, C₁-C₆ perfluoroalkyl, or C₁-C₆ alkoxy; or cyclohexyl, 3-dimethylaminopropyl, or N-morpholinoethyl). ** Acyl azides can also rearrange to isocyanates | | |

The covalent linkage L binds the reactive group Rₓ or conjugated substance So to the compound, either directly (L is a single bond) or with a combination of stable chemical bonds, optionally including single, double, triple or aromatic carbon-carbon bonds, as well as carbon-nitrogen bonds, nitrogen-nitrogen bonds, carbon-oxygen bonds, carbon-sulfur bonds, phosphorus-oxygen bonds, phosphorus-nitrogen bonds, and nitrogen-platinum bonds. L typically includes ether, thioether, carboxamide, sulfonamide, urea, urethane or hydrazine moieties. In one embodiment, the covalent linkage incorporates a platinum atom, such as described in U.S. Patent No. 5,714,327 . Preferred L moieties have 1-20 nonhydrogen atoms selected from the group consisting of C, N, O, P, and S; and are composed of any combination of ether, thioether, amine, ester, carboxamide, sulfonamide, hydrazide bonds and aromatic or heteroaromatic bonds. Preferably L is a combination of single carbon-carbon bonds and carboxamide or thioether bonds. The longest linear segment of the linkage L preferably contains 4-10 nonhydrogen atoms, including one or two heteroatoms. Examples of L include substituted or unsubstituted polymethylene, arylene, alkylarylene, arylenealkyl, or arylthio. In one embodiment, L contains 1-6 carbon atoms; in another, L comprises a thioether linkage. In yet another embodiment, L is or incorporates the formula -(CH₂)_{d}(CONH(CH₂)ₑ)_{z}-, or -(CH₂)_{d}(CON(CH₂)₄NH(CH₂)ₐ)_{z'}-, -(CH₂)_{d}(CONH(CH₂)_{c}NH₂)_{z}'-, -(CH₂)_{d}(CONH(CH₂)ₐNHCO)-_{z}', where d is 0-5, e is 1-5 and z' is 0 or 1.

Choice of the reactive group used to attach the dye to the substance to be conjugated typically depends on the functional group on the substance to be conjugated and the type or length of covalent linkage desired. The types of functional groups typically present on the organic or inorganic substances include, but are not limited to, amines, amides, thiols, alcohols, phenols, aldehydes, ketones, phosphates, imidazoles, hydrazines, hydroxylamines, disubstituted amines, halides, epoxides, carboxylate esters, sulfonate esters, purines, pyrimidines, carboxylic acids, olefinic bonds, or a combination of these groups. A single type of reactive site may be available on the substance (typical for polysaccharides), or a variety of sites may occur (e.g. amines, thiols, alcohols, phenols), as is typical for proteins. A conjugated substance may be conjugated to more than one dye, which may be the same or different, or to a substance that is additionally modified by a hapten, such as biotin. Although some selectivity can be obtained by careful control of the reaction conditions, selectivity of labeling is best obtained by selection of an appropriate reactive dye.

Typically, Rₓ will react with an amine, a thiol, an alcohol, an aldehyde or a ketone. Preferably Rₓ reacts with an amine or a thiol functional group. Rₓ is a reactive amine (including a cadaverine or ethylenediamine), an activated ester of a carboxylic acid (typically a succinimidyl ester of a carboxylic acid), a carboxylic acid or a maleimide.

Where Rₓ is an activated ester of a carboxylic acid, the reactive dye is particularly useful for preparing dye-conjugates of proteins, nucleotides, oligonucleotides, or haptens. Where Rₓ is a maleimide the reactive dye is particularly useful for conjugation to thiol-containing substances.

Based on the above-mentioned attributes, the appropriate reactive dye of the invention is selected for the preparation of the desired dye-conjugate, whose advantageous properties make them useful for a wide variety of applications. When compared to conjugates of widely used carbocyanine dyes for which the point of attachment is at the 1-position of the indolium moiety (e.g. Amersham's Cy dyes^{™}), the dye-conjugates of this invention have demonstrably superior optical properties. The desired dye-conjugate is selected based on the intended application.

Particularly useful dye-conjugates include, among others, conjugates where Sc is an antigen, steroid, vitamin, drug, hapten, metabolite, toxin, environmental pollutant, amino acid, peptide, protein, nucleic acid, nucleic acid polymer, carbohydrate, lipid, ion-complexing moiety, or glass, plastic or other non-biological polymer. Alternatively, Sc is a cell, cellular system, cellular fragment, or subcellular particle, e.g. inter alia, a virus particle, bacterial particle, virus component, biological cell (such as animal cell, plant cell, bacteria, yeast, or protist), or cellular component. Reactive dyes typically label functional groups at the cell surface, in cell membranes, organelles, or cytoplasm.

Typically Sc is an amino acid, peptide, protein, tyramine, polysaccharide, ion-complexing moiety, nucleoside, nucleotide, oligonucleotide, nucleic acid, hapten, psoralen, drug, hormone, lipid, lipid assembly, polymer, polymeric microparticle, biological cell or virus. More typically, Sc is a peptide, a protein, a nucleotide, an oligonucleotide, or a nucleic acid. When conjugating dyes of the invention to such biopolymers, it is possible to incorporate more dyes per molecule to increase the fluorescent signal. For example, it is possible to incorporate at least four molecules of such dyes per molecule of antibody without loss of total fluorescence, whereas fluorescence of the spectrally comparable Cy5 (wherein n = 2) is strongly quenched when greater than approximately two Cy5 dyes are incorporated per antibody. These results confirm problems with Cy dye conjugates reported by others, e.g. BIOCONJUGATE CHEM. 11, 696 (2000). A comparison of commercially available Cy5 conjugates or conjugates of known dyes, and the optimally labeled conjugates of the invention are typically at least two-fold, usually more than three-fold and sometimes more than four-fold more fluorescent than conjugates of the Cy5 dye at the same antibody concentration (Table 3).

| **Table 3** | | | | | |
|---|---|---|---|---|---|
| **Protein** | **Source** | **Compound** | **DOS‡** | **RQY†** | **TF§** |
| **GAR IgG** | Molecular Probes | 9 | 2.10 | 1.6 | 3.3 |
| **GAR IgG** | Molecular Probes | 9 | 3.0 | 1.4 | 4.3 |
| **GAR IgG** | Molecular Probes | 9 | 4.1 | 1.1 | 4.5 |
| **GAR IgG** | Molecular Probes | 9 | 5.2 | 0.9 | 4.7 |
| **GAR IgG** | Molecular Probes | 9 | 7.6 | 0.6 | 4.6 |
| **GAR IgG** | Molecular Probes | 9 | 8.2 | 0.5 | 4.1 |
| **GARIgG** | Molecular Probes | Cy5 | 2.4 | 0.95 | 2.3 |
| **GAR IgG** | Molecular Probes | Cy5 | 4.1 | 0.5 | 2.1 |
| **GAR IgG** | Molecular Probes | Cy5 | 4.5 | 0.2 | 0.9 |
| **GAR IgG** | Molecular-Probes | Cy5 | 5 | 0.3 | 1.5 |
| **GAR IgG** | Molecular Probes | Cy5 | 5.8 | 0.03 | 0.2 |
| **GAR IgG** | Jackson Labs | Cy5 | 2.2 | 1.1 | 2.4 |
| **GAR IgG** | Chemicon | Cy5 | 3.3 | 0.82 | 2.7 |
| **GAR IgG** | Zymed | Cy5 | 4.7 | 0.53 | 2.5 |
| **GAR IgG** | Amersham-Pharmacia Biotech | Cy5 | 5.7 | 0.22 | 1.3 |
| **GAR IgG** | Kirkegaard & Perry | Cy5 | 5.7 | 0.20 | 1.1 |
| **GAR IgG** | Rockland | Cy5 | 10.1 | 0.05 | 0.5 |
| **GAR IgG** | Molecular Probes | 30 | 2.7 | 0.7 | 1.9 |
| **GAR IgG** | Molecular Probes | 30 | 4.3 | 0.28 | 1.2 |
| **GAR IgG** | Molecular Probes | 30 | 5.4 | 0.10 | 0.5 |
| **GAR IgG** | Molecular Probes | 24 | 2.9 | 0.8 | 2.2 |
| **GAR IgG** | Molecular Probes | 24 | 4.3 | 0.33 | 1.4 |
| **GAR IgG** | Molecular Probes | 24 | 5.6 | 0.15 | 0.8 |
| **GAR IgG** | Molecular Probes | 25 | 2.0 | 0.7 | 1.4 |
| **GAR IgG** | Molecular Probes | 25 | 2.9 | 0.35 | 1.0 |
| **GAR IgG** | Molecular Probes | 25 | 3.9 | 0.13 | 0.5 |
| **GAR IgG** | Molecular Probes | 27 | 2.0 | 0.9 | 1.8 |
| **GAR IgG** | Molecular Probes | 27 | 3.2 | 0.52 | 1.6 |
| **GAR IgG** | Molecular Probes | 27 | 4.2 | 0.28 | 1.2 |
| **GAR IgG** | Molecular Probes | 26 | 1.4 | 0.58 | 0.81 |
| **GAR IgG** | Molecular Probes | 26 | 2.1 | 0.8 | 0.61 |
| **GAR IgG** | Molecular Probes | 26 | 2.9 | 0.1 | 0.29 |
| | | | | | |
| **GAM IgG** | Molecular Probes | 9 | 2.1 | 1.4 | 2.9 |
| **GAM IgG** | Molecular Probes | 9 | 2.2 | 1.4 | 3.1 |
| **GAM IgG** | Molecular Probes | 9 | 3.1 | 1.1 | 8.5 |
| **GAM IgG** | Molecular Probes | 9 | 4.2 | 1.2 | 5.0 |
| **GAM IgG** | Molecular Probes | 9 | 5.2 | 0.6 | 3.2 |
| **GAM IgG** | Jackson Labs | Cy5 | 1.9 | 1.0 | 1.6 |
| **GAM IgG** | Molecular Probes | Cy5 | 2 | 0.9 | 1.8 |
| **GAM IgG** | Molecular Probes | Cy5 | 8.3 | 0.5 | 1.6 |
| **GAM IgG** | Molecular Probes | Cy5 | 4.8 | 0.09 | 0.4 |
| | | | | | |
| **Concanavalin A** | Molecular Probes | 9 | 1.7 | 1.2 | 2.0 |
| **Concanavalin A** | Molecular Probes | 9 | 2.2 | 0.8 | 1.8 |
| **Concanavalin A** | Molecular Probes | 9 | 3.3 | 0.9 | 2.9 |
| **Concanavalin A** | Molecular Probes | 9 | 3.7 | 0.6 | 2.3 |
| **Concanavalin A** | Molecular Probes | 9 | 5.5 | 0.6 | 3.3 |
| **Concanavalin A** | Molecular Probes | 9 | 5.6 | 0.8 | 4.5 |
| **Concanavalin A** | Molecular Probes | Oy5 | 1.3 | 0.9 | 1.2 |
| **Concanavalin A** | Molecular Probes | Cy5 | 2.4 | 0.5 | 1.2 |
| **Concanavalin A** | Molecular Probes | Cy5 | 3.1 | 0.2 | 0.6 |
| **Concanavalin A** | Molecular Probes | Cy5 | 3.3 | 0.8 | 2.6 |
| **Concanavalin A** | Molecular Probes | Cy5 | 4.4 | 0.3 | 1.3 |
| **Concanavalin A** | Molecular Probes | Cy5 | 6.5 | 0.1 | 0.7 |
| | | | | | |
| **Streptavidin** | Molecular Probes | 9 | 2.2 | 2.4 | 5.4 |
| **Streptavidin** | Molecular Probes | 9 | 2.8 | 1.9 | 5.3 |
| **Streptavidin** | Molecular Probes | 9 | 3.4 | 1.9 | 6.5 |
| **Streptavidin** | Molecular Probes | 9 | 4.1 | 1.8 | 7.3 |
| **Streptavidin** | Molecular Probes | 9 | 4.5 | 1.7 | 7.6 |
| **Streptavidin** | Molecular Probes | 9 | 5.2 | 1.6 | 8.3 |
| **Streptavidin** | Molecular Probes | Cy5 | 1.6 | 1.8 | 2.8 |
| **Streptavidin** | Molecular Probes | Cy5 | 2.7 | 1.5 | 4.1 |
| **Streptavidin** | Jackson Labs | Cy5 | 3.3 | 1.8 | 6.0 |
| **Streptavidin** | Amersham-Pharmacia Biotech | Cy5 | 3.6 | 1.4 | 5.0 |
| **Streptavidin** | Molecular Probes | Cy5 | 3.6 | 1.4 | 5.0 |
| | | | | | |
| **Transferrin** | Molecular Probes | 9 | 1.8 | 1.5 | 2.7 |
| **Transferrin** | Molecular Probes | 9 | 2.7 | 1.3 | 3.5 |
| **Transferrin** | Molecular Probes | 9 | 3.7 | 1.2 | 4.4 |
| **Transferrin** | Molecular Probes | 9 | 4.2 | 1.1 | 4.4 |
| **Transferrin** | Molecular Probes | 9 | 5.8 | 0.7 | 4.1 |
| **Transferrin** | Molecular Probes | 9 | 5.9 | 0.6 | 3.5 |
| **Transferrin** | Molecular Probes | Cy5 | 0.8 | 1.1 | 0.9 |
| **Transferrin** | Molecular Probes | Cy5 | 1.4 | 0.8 | 1.1 |
| **Transferrin** | Molecular Probes | Cy5 | 2.7 | 0.5 | 1.4 |
| **Transferrin** | Molecular Probes | Cy5 | 3.0 | 0.7 | 2.1 |
| **Transferrin** | Molecular Probes | Cy5 | 5.3 | 0.1 | 0.5 |
| **Transferrin** | Molecular Probes | Cy5 | 5.8 | 0.02 | 0.1 |

| | | | | | |
|---|---|---|---|---|---|
| †RQY (Relative Quantum Yield) is measured by matching absorbance between bioconjugate and DDAO (7-hydroxy-9H-(1,3-dichloro-9,9-dimethylacridine-2-one)), and comparing integrated fluorescence emission intensities. ‡DOS (Degree of Substitution) is the approximate molar ratio of the dye to protein following conjugation. DOS estimated using Molar Extinction coefficients of 250,000 M⁻¹ cm⁻¹ for Cy5 and 239,000 M⁻¹ cm⁻¹ for Compound 9 bioconjugates respectively, and protein extinction coefficients from the literature and a visible dye-correction term (at 280 nm) of 3-5%. §TF (Total Fluorescence) is proportional to the overall brightness of the bioconjugate, and is defined as the product of the RQY and DOS: TF=RQY x DOS. Compounds 9, 25-27 are reference dyes, Cy5 and Compound 30 are comparative dyes. | | | | | |

Alternatively, Sc is a ligand or a hapten, such as biotin. A preferred conjugate is a phenol such as a tyramine (e.g. as described in U.S. Patents 5,196,306; 5,583,001; 5,731,158 ), wherein the conjugate is useful as a substrate for horseradish peroxidase.

In one embodiment, Sc is a biological polymer such as a peptide, protein, oligonucleotide, or nucleic acid polymer that is also labeled with at least a second non-fluorescent or fluorescent dye (optionally an additional dye of the present invention), to form an energy-transfer pair. In some aspects of the invention, the labeled conjugate functions as an enzyme substrate, and enzymatic hydrolysis disrupts the energy transfer. Alternatively, Sc is itself a fluorescent or nonfluorescent dye, optionally an additional dye of the present invention, which dye-conjugate forms a labeling complex that exhibits a large Stokes shift due to internal energy-transfer (as described in U.S. Pat. 6,008,373 above), which complex is useful to label an organic or inorganic substance (Example 52).

In one embodiment, Sc is an amino acid (including those that are protected or are substituted by phosphates, carbohydrates, or C₁ to C₂₂ carboxylic acids), or is a polymer of amino acids such as a peptide or protein. Preferred conjugates of peptides contain at least five amino acids, more preferably 5 to 36 amino acids. Preferred peptides include, but are not limited to, neuropeptides, cytokines, toxins, protease substrates, and protein kinase substrates. Preferred protein conjugates include enzymes, antibodies, lectins, glycoproteins, histones, albumins, lipoproteins, avidin, streptavidin, protein A, protein G, phycobiliproteins and other fluorescent proteins, hormones, toxins, chemokines and growth factors. In one preferred aspect, the conjugated protein is a phycobiliprotein, such as allophycocyanin, phycocyanin, phycoerythrin, allophycocyanin B, B-phycoerythrin, phycoerythrocyanin, and b-phycoerythrin (for example, see U.S. Pat. 5,714,386 to Roederer (1998). ). Particularly preferred are conjugates of R-phycoerythrin and of allophycocyanin with selected dyes of the invention that serve as excited-state energy acceptors or donors. In these conjugates, excited state energy transfer results in long wavelength fluorescence emission when excited at relatively short wavelengths (Example 52). In another aspect of the invention, the conjugated protein is an antibody, an antibody fragment, avidin, streptavidin, a toxin, a lectin, a hormone, a chemokine, or a growth factor. Typically, where the conjugated substance is a toxin, it is a neuropeptide or a phallotoxin, such as phalloidin.

In another embodiment, So is a nucleic add base, nucleoside, nucleotide (Example 55) or a nucleic acid polymer (Examples 56-59), including those that are modified to possess an additional linker or spacer for attachment of the dyes of the invention, such as an alkynyl linkage (U.S. Pat. 5,047,519), an aminoallyl linkage (U.S. Pat. 4,711,955), or a heteroatom-substituted linker (U.S. Pat. 5,684,142), or other linkage. In another embodiment, the conjugated substance is a nucleoside or nucleotide analog that links a purine or pyrimidine base to a phosphate or polyphosphate moiety through a noncyclic spacer. In a third embodiment, the dye is conjugated to the carbohydrate portion of a nucleotide or nucleoside, typically through a hydroxyl group but additionally through a thiol or amino group (U.S. Pat. 5,659,025, U.S. Pat. 5,668,268, U.S. Pat. 5,679,785 ). Typically, the conjugated nucleotide is a nucleoside triphosphate or a deoxynucleoside triphosphate or a dideoxynucleoside triphosphate. Incorporation of methylene moieties or nitrogen or sulfur heteroatoms into the phosphate or polyphosphate moiety is also useful. Nonpurine and nonpyrimidine bases such as 7-deazapurines (US Pat. 6,150,510) and nucleic acids containing such bases can also be coupled to dyes of the invention. Nucleic acid adducts prepared by reaction of depurinated nucleic acids with amine, hydrazide or hydroxylamine derivatives provide an additional means of labeling and detecting nucleic acids, e.g. "A method for detecting abasic sites in living cells: age-dependent changes in base excision repair." Atamna H, Cheung I, Ames BN. Proc NatI Acad Sci U S A 97, 686-691 (2000).

Preferred nucleic acid polymer conjugates are labeled, single- or multi-stranded, natural or synthetic DNA or RNA, DNA or RNA oligonucleotides, or DNA/RNA hybrids, or incorporate an unusual linker such as morpholine derivatized phosphates (AntiVirals, Inc., Corvallis OR), or peptide nucleic acids such as *N*-(2-aminoethyl)glycine units. When the nucleic acid is a synthetic oligonucleotide, it typically contains fewer than 50 nucleotides, more typically fewer than 25 nucleotides. Conjugates of peptide nucleic acids (PNA) (Nielsen et al U.S. Pat. 5,539,082) may be preferred for some applications because of their generally faster hybridization rates.

Fluorescent nucleic acid polymers are typically prepared from labeled nucleotides or oligonucleotides using oligonucleotide-primed DNA polymerization (Example 59), such as by using the polymerase chain reaction or through primer extension, or by terminal-transferase catalyzed addition of a labeled nucleotide to a 3 end of a nucleic acid polymer. Fluorescent RNA polymers are typically prepared from labeled nucleotides by transcription. Typically, the dye is attached via one or more purine or pyrimidine bases through an amide, ester, ether or thioether bond; or is attached to the phosphate or carbohydrate by a bond that is an ester, thioester, amide, ether or thioether. Alternatively, dye conjugate of the invention is simultaneously labeled with a hapten such as biotin or digoxigenin, or to an enzyme such as alkaline phosphatase, or to a protein such as an antibody. Nucleotide conjugates of the invention are readily incorporated by DNA polymerase and can be used for *in situ* hybridization and nucleic acid sequencing (e.g., U.S. Patents 5,332,666; 5,171,534; and 4,997,928, and WO Appl. 94/05688). In another aspect of the invention, the oligonucleotide incorporates an aliphatic amine, which is subsequently conjugated to an amine-reactive dye of the invention or a thiol or thiophosphate, which is conjugated to a thiol-reactive dye of the invention. In yet another aspect of the invention, the purine bases of the oligonucleotide react with a reactive metal complex (preferably a platinum complex) bound to a dye of the invention, yielding a dye-conjugate (Example 58). Nucleic acid conjugates of dyes of the invention that are linked at the 3-position of the indolium ring unexpectedly have spectral properties that are superior to those of structurally similar carbocyanine dyes wherein the dye is not linked at the 3-position of the indolium ring (Examples 58-60, Table 8).

In one embodiment, the conjugated oligonucleotides of the invention are aptamers for a particular target molecule, such as a metabolite, dye, hapten, or protein. That is, the oligonucleotides have been selected to bind preferentially to the target molecule. Methods of preparing and screening aptamers for a given target molecule have been previously described and are known in the art (for example, U.S. Pat. 5,567,588 to Gold (1996)).

In another embodiment, the conjugated substance (S_{c}) is a carbohydrate that is typically a polysaccharide, such as a dextran, FICOLL^{™}, heparin, glycogen, amylopectin, mannan, inulin, starch, agarose and cellulose. Alternatively, the carbohydrate is a polysaccharide that is a lipopolysaccharide. Preferred polysaccharide conjugates are dextran, FICOLL^{™}, or lipopolysaccharide conjugates.

In another embodiment, the conjugated substance (S_{c}), is a lipid (typically having 6-60 carbons), including glycolipids, phospholipids, sphingolipids, and steroids. Alternatively, the conjugated substance is a lipid assembly, such as a liposome. The lipophilic moiety may be used to retain the conjugated substances in cells, as described in US Pat. 5,208,148 . Certain polar dyes of the invention may also be trapped within lipid assemblies.

Conjugates having an ion-complexing moiety serve as indicators for calcium, sodium, magnesium, zinc, potassium, or other biologically important metal ions. Preferred ion-complexing moieties are crown ethers (U.S. Pat. 5,405,975 ); derivatives of 1,2-bis-(2-aminophenoxyethane)-*N,N,N',N'*-tetraacetic acid (BAPTA chelators; U.S. Pat. 5,453,517, U.S. Pat. 5,516,911, and U.S. Pat. 5,049,673. ); derivatives of 2-carboxymethoxyaniline-N,N-diacetic acid (APTRA chelators; AM. J. PHYSIOL. 256, C540 (1989), incorporated by reference); or pyridine- and phenanthroline-based metal ion chelators (U.S. Pat. 5,648,270, incorporated by reference); or derivatives of nitrilotriacetic acid, see e.g. "Single-step synthesis and characterization of biotinylated nitrilotriacetic acid, a unique reagent for the detection of histidine-tagged proteins immobilized on nitrocellulose". McMahan SA, Burgess RR. Anal Biochem 236, 101-106 (1996). Preferably, the ion-complexing moiety is a crown ether chelator, a BAPTA chelator, an APTRA chelator or a derivative of nitrilotriacetic acid.

Other conjugates of non-biological materials include dye-conjugates of organic or inorganic polymers, polymeric films, polymeric wafers, polymeric membranes, polymeric particles, or polymeric microparticles (Example 53); including magnetic and non-magnetic microspheres; iron, gold or silver particles; conducting and nonconducting metals and non-metals; and glass and plastic surfaces and particles. Conjugates are optionally prepared by copolymerization of a dye that contains an appropriate functionality while preparing the polymer, or by chemical modification of a polymer that contains functional groups with suitable chemical reactivity. Other types of reactions that are useful for preparing dye-conjugates of polymers include catalyzed polymerizations or copolymerizations of alkenes and reactions of dienes with dienophiles, transesterifications or transaminations. In another embodiment, the conjugated substance is a glass or silica, which may be formed into an optical fiber or other structure.

In one aspect of the invention, S_{c} is a conjugated substance that is an antibody (including intact antibodies, antibody fragments, and antibody sera, etc.), an amino acid, an angiostatin or endostatin, an avidin or streptavidin, a biotin (e.g. an amidobiotin, a biocytin, a desthiobiotin, etc.), a blood component protein (e.g. an albumin, a fibrinogen, a plasminogen, etc.), a dextran, an enzyme, an enzyme inhibitor, an IgG-binding protein (e.g. a protein A, protein G, protein A/G, etc.), a fluorescent protein (e.g. a phycobiliprotein, an aequorin, a green fluorescent protein, etc,), a growth factor, a hormone, a lectin (e.g. a wheat germ agglutinin, a conconavalin A, etc.), a lipopolysaccharide, a metal-binding protein (e.g. a calmodulin, etc.), a microorganism or portion thereof (e.g. a bacteria, a virus, a yeast, etc.), a neuropeptide and other biologically active factors (e,g. a dermorphin, a deltropin, an endomorphin, an endorphin, a tumor necrosis factor etc.), a non-biological microparticle (e.g. of ferrofluid, gold, polystyrene, etc.), a nucleotide, an oligonucleotide, a peptide toxin (e.g. an apamin, a bungarotoxin, a phalloidin, etc.), a phospholipid-binding protein (e,g. an annexin etc.), a small-molecule drug (e.g, a methotrexate, etc.), a structural protein (e.g. an actin, a fibronectin, a laminin, a microtubule-associated protein, a tublin, etc.), or a tyramide.

In one embodiment, conjugates of biological polymers such as peptides, proteins, oligonucleotides, nucleic acid polymers are also labeled with at least a second fluorescent or nonfluereseent dye, that is optionally an additional dye of the present invention, to form an energy-trausfer pair. In some aspects of the invention, the labeled conjugate functions as an enzyme substrate, and enzymatic hydrolysis disrupts the energy transfer. Alternatively, the conjugated substance is itself a fluorescent or nontluorescent dye, optionally an additional dye of the present invention, that forms a labeling complex that exhibits a large Stokes shift due to internal energy-transfer (as described in U.S. Pat. 6,008,373 to Waggoner et al., (1999)). In another embodiment of the invention, the energy-transfer pair that incorporates a dye of the invention is conjugated to an oligonucleotide that displays efficient fluorescence quenching in its hairpin conformation (the so-called "molecular beacons" of Tyagi et aL, NATURE BIOTECHNOLOGY 16, 49 (1998) ) or fluorescence energy transfer.

The preparation of dye conjugates using reactive dyes is well documented, e.g. by R. Haugland, MOLECULAR PROBES HANDBOOK OF FLUORESCENT PROBES AND RESEARCH CHEMICALS, Chapters 1-3 (1996); and Brinkley, BIOCONJUGATE CHEM., 3, 2 (1992). Conjugates typically result from mixing appropriate reactive dyes and the substance to be conjugated in a suitable solvent in which both are soluble. The majority of the dyes of the invention are readily soluble in aqueous solutions, facilitating conjugation reactions with most biological materials. For those reactive dyes that are photoactivated, conjugation requires illumination of the reaction mixture to activate the reactive dye.

Labeled members of a specific binding pair are typically used as fluorescent probes for the complementary member of that specific binding pair, each specific binding pair member having an area on the surface or in a cavity that specifically binds to and is complementary with a particular spatial and polar organization of the other. Preferred specific binding pair members are proteins that bind non-covalently to low molecular weight ligands, such as biotin, drug-haptens and fluorescent dyes (such as an anti-fluorescein antibody). Representative specific binding pairs are shown in Table 4.

**Table 4. Representative Specific Binding Pairs**

| | |
|---|---|
| antigen | antibody |
| biotin | avidin (or streptavidin or antibiotin) |
| IgG* | protein A or protein G |
| drug | drug receptor |
| toxin, | toxin receptor |
| carbohydrate | lectin or carbohydrate receptor |
| peptide | peptide receptor |
| protein | protein receptor |
| enzyme substrate | enzyme |
| DNA (RNA) | aDNA (aRNA)† |
| hormone | hormone receptor |
| ion | chelator |
| psoralen | nucleic acid |
| target molecule | RNA or DNA aptamer |

| | |
|---|---|
| * IgG is an immunoglobulin † aDNA and aRNA are the antisense (complementary) strands used for hybridization | |

In one aspect of the invention, the conjugated substance is further labeled with additional dye moieties, such that fluorescence energy is either accepted from, or transferred to, the dye of the invention.

### Synthesis

Synthesis of the carbocyanine dyes of the invention, where attachment is at the 3-position of the indolium, depends on initial preparation of certain key intermediates. The intermediates have the following general structure (for simplicity, all but a few of the possible substituents are shown as hydrogen):

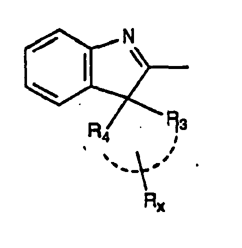

This basic structure is optionally further substituted, during or after synthesis, to give the corresponding dye substituents as defined above.

The novel key intermediates are readily synthesized by a reaction that is analogous to a Fischer indole synthesis (where X is a desired substituent on the resulting indolium, typically sulfo, and R⁸ and R⁴ are as defined above): 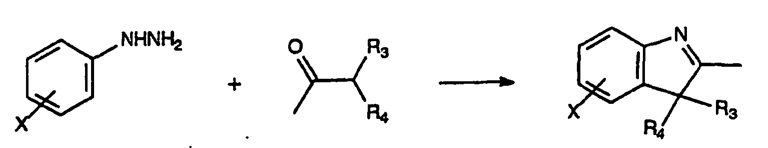 In this reaction, an appropriately substituted aryl hydrazine, which is typically a phenylhydrazine, is reacted with an appropriately substituted methyl ketone to yield a 3,3-disubstituted 2-methylindole derivative. One of the 3-position substituents is selected to be a chemically reactive moiety or a group that is converted to a chemically reactive moiety such as a carboxylic acid derivative (Examples 1,2,7), an alcohol (Example 16) or an amine (Example 17), It is particularly suitable to utilize a sulfonated phenylhydrazine derivative (as in Examples 1-3) to increase the solubility of the final dye. The 3,3-disubstituted-2-methylindole is then quaternized on the nitrogen atom to an indolium derivative with an alkylating agent that is typically an alkyl halide such as ethyl iodide, an alkylsulfonate such as methyl p-toluemesulfonate (Example 7) or a cyclic sulfonate such as propanesultone or butanesultone (Examples 2-3). Typically, the key indolium intermediates are sulfonated one or more times before or after quaternization at R² and subsequent condensation with the benzazolium moiety and polymethine moiety to form the subject dyes. Methods for synthesis of dyes wherein n = 1, n = 2 and n = 3 are provided in Examples 12, 8, and 21, respectively.

The bridge moiety typically originates from the coupling agent used in the dye construction.

The methods for synthesis of dyes that contain a variety of reactive groups such as those described in Table 2 are well documented in the art. Particularly useful are amine-reactive dyes such as "activated esters" of carboxylic acids, which are typically synthesized by coupling a carboxylic acid to a relatively acidic "leaving group".

Nucleosides and nucleotides labeled with dyes of the invention are particularly useful for some applications of nucleic acid labeling. The use of carbocyanine-amidites for labeling nucleotides and nucleosides have been previously described (U.S. 5,986,086 to Bruch et al. (1999); U.S. 5,808,044 to Brush et al. (1998); U.S. 5,556,959 to Brush et al. (1996); ).

Examples of some synthetic strategies for selected dyes, as well as their characterization, synthetic precursors, conjugates and method of use are provided in the examples below. Further modifications and permutations will be obvious to one skilled in the art.

### Applications and Methods of Use

In one aspect of the disclosure, the dye compounds of the invention are used to directly stain or label a sample so that the sample can be identified or quantitated. For instance, such dyes may be added as part of an assay for a biological target analyte, as a detectable tracer element in a biological or non-biological fluid, or for such purposes as photodynamic therapy of tumors, in which a dyed sample is irradiated to selectively destroy tumor cells and tissues, or to photoablate arterial plaque or cells, usually through the photosensitized production of singlet oxygen. In one preferred embodiment, dye conjugate is used to stain a sample that comprises a ligand for which the conjugated substance is a complementary member of a specific binding pair (e.g. Table 4).

In one aspect of the disclosure, the sample is obtained directly from a liquid source or as a wash from a solid material (organic or inorganic) or a growth medium in which cells have been introduced for culturing, or a buffer solution in which cells have been placed for evaluation. Where the sample comprises cells, the cells are optionally single cells, including microorganisms, or multiple cells associated with other cells in two or three dimensional layers, including multicellular organisms, embryos, tissues, biopsies, filaments, biofilms, etc.

Alternatively, the sample is a solid, optionally a smear or scrape or a retentate removed from a liquid or vapor by filtration. In one aspect of the disclosure, the sample is obtained from a biological fluid, including separated or unfiltered biological fluids such as urine, cerebrospinal fluid, blood, lymph fluids, tissue homogenate, interstitial fluid, cell extracts, mucus, saliva, sputum, stool, physiological secretions or other similar fluids. Alternatively, the sample is obtained from an environmental source such as soil, water, or air; or from an industrial source such as taken from a waste stream, a water source, a supply line, or a production lot.

In yet another embodiment, the sample is present on or in solid or semi-solid matrix. In one aspect of the disclosure, the matrix is a membrane. In another aspect, the matrix is an electrophoretic gel, such as is used for separating and characterizing nucleic acids or proteins, or is a blot prepared by transfer from an electrophoretic gel to a membrane. In another aspect, the matrix is a silicon chip or glass slide, and the analyte of interest has been immobilized on the chip or slide in an array (e.g. the sample comprises proteins or nucleic acid polymers in a microarray). In yet another aspect, the matrix is a microwell plate or microfluidic chip, and the sample is analyzed by automated methods, typically by various methods of high-throughput screening, such as drug screening.

The dye compounds of the invention are generally utilized by combining a dye compound of the invention as described above with the sample of interest under conditions selected to yield a detectable optical response. The term "dye compound" is used herein to refer to all aspects of the claimed dyes, including both reactive dyes and dye conjugates. The dye compound typically forms a covalent or non-covalent association or complex with an element of the sample, or is simply present within the bounds of the sample or portion of the sample. The sample is then illuminated at a wavelength selected to elicit the optical response. Typically, staining the sample is used to determine a specified characteristic of the sample by further comparing the optical response with a standard or expected response.

A detectable optical response means a change in, or occurrence of, an optical signal that is detectable either by observation or instrumentally. Typically the detectable response is a change in fluorescence, such as a change in the intensity, excitation or emission wavelength distribution of fluorescence, fluorescence lifetime, fluorescence polarization, or a combination thereof. The degree and/or location of staining, compared with a standard or expected response, indicates whether and to what degree the sample possesses a given characteristic. Some dyes of the invention may exhibit little fluorescence emission, but are still useful as chromophoric dyes. Such chromophores are useful as energy acceptors in FRET applications, or to simply impart the desired color to a sample or portion of a sample.

For biological applications, the dye compounds of the invention are typically used in an aqueous, mostly aqueous or aqueous-miseible solution prepared according to methods generally known in the art. The exact concentration of dye compound is dependent upon the experimental conditions and the desired results, but typically ranges from about one nanomolar to one millimolar or more. The optimal concentration is determined by systematic variation until satisfactory results with minimal background fluorescence is accomplished.

The dye compounds are most advantageously used to stain samples with biological components. The sample may comprise heterogeneous mixtures of components (including intact cells, cell extracts, bacteria, viruses, organelles, and mixtures thereof), or a single component or homogeneous group of components (e.g. natural or synthetic amino acid, nucleic acid or carbohydrate polymers, or lipid membrane complexes). These dyes are generally non-toxic to living cells and other biological components, within the concentrations of use.

The dye compound is combined with the sample in any way that facilitates contact between the dye compound and the sample components of interest. Typically, the dye compound or a solution containing the dye compound is simply added to the sample. Certain dyes of the invention, particularly those that are substituted by one or more sulfonic acid moieties, tend to be impermeant to membranes of biological cells, and once inside viable cells are typically well retained. Treatments that permeabilize the plasma membrane, such as electroporation, shock treatments or high extracellular ATP can be used to introduce selected dye compounds into cells. Alternatively, selected dye compounds can be physically inserted into cells, e.g. by pressure microinjection, scrape loading, patch damp methods, or phagocytosis.

Chemically reactive dye compounds will covalently attach to a corresponding functional group on a wide variety of materials, forming dye conjugates as described above. Using dye compounds to label reactive sites on the surface of cells, in cell membranes or in intracellular compartments such as organelles, or in the cell's cytoplasm, permits the determination of their presence or quantity, accessibility, or their spatial and temporal distribution in the sample.

Optionally, the sample is washed after staining to remove residual, excess or unbound dye compound. The sample is optionally combined with one or more other solutions in the course of staining, including wash solutions, permeabilization and/or fixation solutions, and solutions containing additional detection reagents. An additional detection reagent typically produces a detectable response due to the presence of a specific cell component, intracellular substance, or cellular condition, according to methods generally known in the art. Where the additional detection reagent has, or yields a product with, spectral properties that differ from those of the subject dye compounds, multi-color applications are possible. This is particularly useful where the additional detection reagent is a dye or dye-conjugate of the present invention having spectral properties that are detectably distinct from those of the staining dye.

The compounds of the invention that are dye conjugates are used according to methods extensively known in the art; e.g. use of antibody conjugates in microscopy and immunofluorescent assays; and nucleotide or oligonucleotide conjugates for nucleic acid hybridization assays and nucleic acid sequencing (e.g., US Patent Nos. 5,332,666 to Prober, et al. (1994); 5,171,534 to Smith, et al. (1992); 4,997,928 to Hobbs (1991); and WO Appl. 94/05688 to Menchen, et al..). Dye-conjugates of multiple independent dyes of the invention possess utility for multi-color applications.

At any time after or during staining, the sample is illuminated with a wavelength of light selected to give a detectable optical response, and observed with a means for detecting the optical response. Equipment that is useful for illuminating the dye compounds of the invention includes, but is not limited to, hand-held ultraviolet lamps, mercury arc lamps, xenon lamps, lasers and laser diodes. These illumination sources are optionally integrated into laser scanners, fluorescence microplate readers, standard or minifluorometers, or chromatographic detectors. Preferred embodiments of the invention are dyes that are be excitable at or near the wavelengths 633-636 nm, 647 nm, 660 nm, 680 nm and beyond 700 nm, as these regions closely match the output of relatively inexpensive excitation sources.

The optical response is optionally detected by visual inspection, or by use of any of the following devices: CCD cameras, video cameras, photographic film, laser-scanning devices, fluorometers, photodiodes, quantum counters, epifluorescence microscopes, scanning microscopes, flow cytometers, fluorescence microplate readers, or by means for amplifying the signal such as photomultiplier tubes. Where the sample is examined using a flow cytometer, examination of the sample optionally includes sorting portions of the sample according to their fluorescence response.

### Kits

One aspect of the instant disclosure is the formulation of kits that facilitate the practice of various assays using any of the dyes of the invention, as described above. The kits of the invention typically comprise a colored or fluorescent dye of the invention, either present as a chemically reactive label useful for preparing dye-conjugates, or present as a dye-conjugate where the conjugated substance is a specific binding pair member, or a nucleoside, nucleotide, oligonucleotide, nucleic acid polymer, peptide, or protein. The kit optionally further comprises one or more buffering agents, typically present as an aqueous solution. The kits of the disclosure optionally further comprise additional detection reagents, a purification medium for purifying the resulting labeled substance, luminescence standards, enzymes, enzyme inhibitors, organic solvent, or instructions for carrying out an assay of the invention.

The examples below are given so as to illustrate the practice of this invention. They are not intended to limit or define the entire scope of this invention.

Examples 2, 5, 8-14, 16-23, 25-32 and 34 - 43 do not form part of the invention as claimed. However, they are retained as useful background information for the reader.

### Example 1. Preparation of 3-(5-carboxypentyl)-2,3-dimethyl-5-sulfoindolium, inner salt (Compound 1).

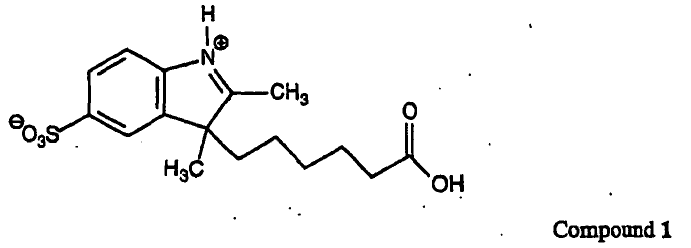

A mixture of 25 g of ethyl 2-methylacetoacetate, 64 mL of a 21 % sodium ethoxide solution in ethanol and 34 mL of ethyl 6-bromohexanoate is relaxed in 200 mL of ethanol overnight. The mixture is filtered and solvent is evaporated. The residue is partitioned between 1 M HCl and chloroform. The organic layer is dried over magnesium sulfate and purified on silica gel using 1:10 ethyl acetate/ hexanes as eluant to yield 22 g of ethyl 2-(5-carbethoxypentyl)-2-methylacetoacetate.

The acetoacetate thus obtained is dissolved in 300 mL of methanol. A solution of 10 g NaOH in 100 mL water is added. The mixture is heated at 50°C overnight. The solution is reduced to ~50 mL, acidified to ~pH 1, and extracted with ethyl acetate. The organic layer is dried over MgSO₄and evaporated to yield 13.5 g of 7-methyl-8-oxononanoic acid. The nonanoic acid is refluxed in 110 mL of acetic acid with 13.5 g of 4-hydrazinobenzenesulfonic acid for 5 hours. The acetic acid is evaporated and the product is purified on silica gel to yield 23 g of the product.

### Example 2. Preparation of 2,3-dimethyl-3(5-carboxyventyl)-5-sulfo-1-(3-sulfopropyl)indolium, sodium salt (Compound 2). (Reference)

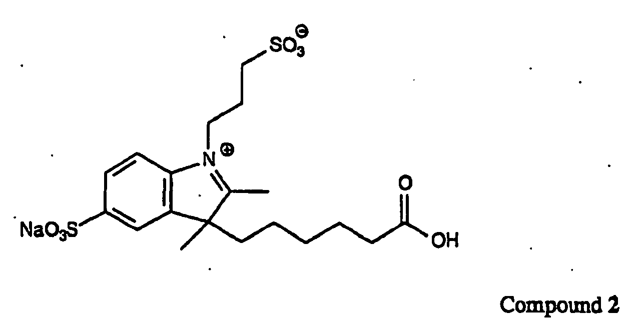

To a methanol solution of 11 g of Compound 1 is added 3.4 g of anhydrous sodium acetate. The mixture is stirred for five minutes. The solvent is evaporated. The resulting sodium salt is heated with 24.4 g of propanesultone at 110°C for 1 hour to generate the product.

### Example 3. Preparation of 5-sulfo-1-(3-sulfopropyl)-2,3,3-trimethylindolium sodium salt (Compound 3A) and 5-sulfo-1-(3-sulfopropyl-1,2,3,3-tetramethylindolium sodium salt (Compound 3B). (Reference)

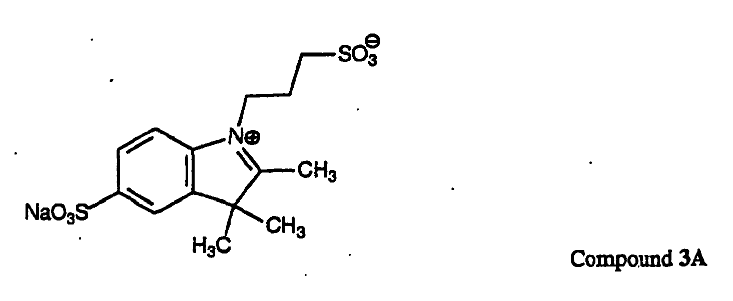

To 15 g of 5-sulfo-2,3,3-trimethylindolium, inner salt (Mujumdar, et al BIOCONJUGATE CHEMISTRY 4,105 (1993)) in 60 mL of methanol is added 5.67 g sodium acetate. After 5 minutes at room temperature, the solution is evaporated. The foamy solid is pulverized, dissolved in 60 mL acetonitrile and stirred with 23 g propanesultone for 15 min. Following evaporation of the solvent, the residue is dried at 110°C to yield 5-sulfo-1-(3-sulfopropyl)-2,3,3-trimethylindolium, sodium salt (Compound 3A). 5-sulfo-1,2,3,3-tetramethylindolium (Compound **3B**) is prepared similarly except that methyl *p-*toluenesulfonate is used instead of propanesultone.

### Example 4. Preparation of 2-(4-anilinobutadienyl)-3,3-dimethyl-5-sulfo-1-(3-sulfopropyl)indolium, sodium salt (Compound 4). (Reference)

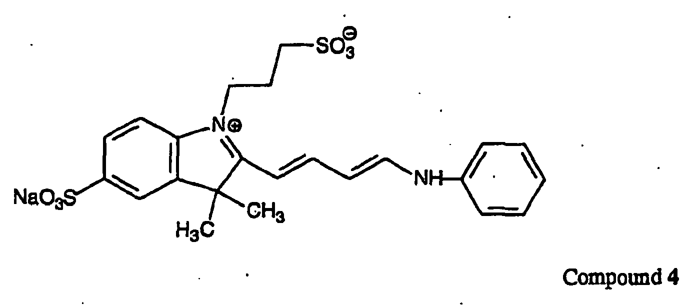

Compound 3A (15 g) is heated with 21.5 g malonaldehyde dianil hydrochloride and 0.4 mL triethylamine in 200 mL of acetic acid at 110°C for one hour. The solvent is evaporated and the residue is purified on silica gel to yield 139 g of the product.

### Example 5. Preparation of 2-(anilinovinyl)-3.3-dimethyl-5-sulfo-1-(3-sulfopropyl)indolium, sodium salt (Compound 5). (Reference)

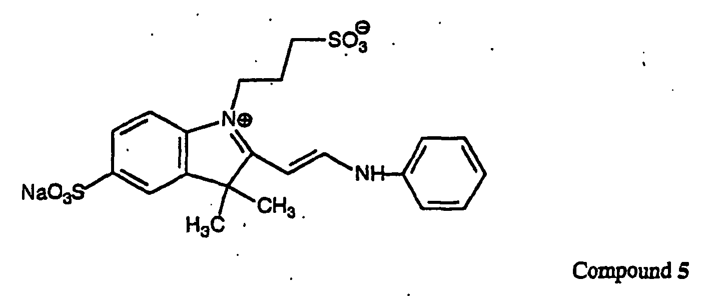

A mixture of 5 g of Compound 3A, 2.72 g of N,N'-diphenylformamidine and 0.52 mL of acetic anhydride is heated at 150°C for 30 minutes, then evaporated and the residue purified on silica gel.

### Example 6. Preparation of 2-(4-anilinobutadienyl)-5-sulfo-1,3,3-trimethylindolium inner salt (Compound 6).

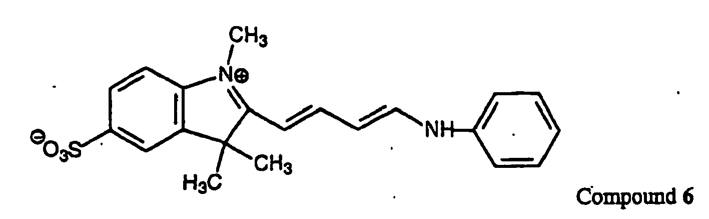

The procedure is the same as used to prepare Compound 4, except that Compound 3B is used instead of Compound 3A.

### Example 7. Preparation of 3-(5-carboxypentyl)-5-sulfo-1,2,3-trimethylindolium, inner salt (Compound 7).

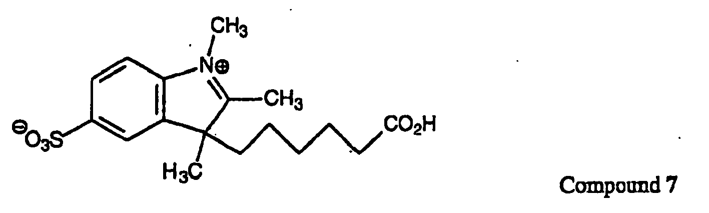

The compound is prepared by heating Compound 1 with 6 equivalents of methyl ptoluenesulfonate-at 100°C for 1.5 hours. The crude product is precipitated with ethyl acetate and purified on a silica gel column.

### Example 8. Preparation of Compound 8. (Reference)

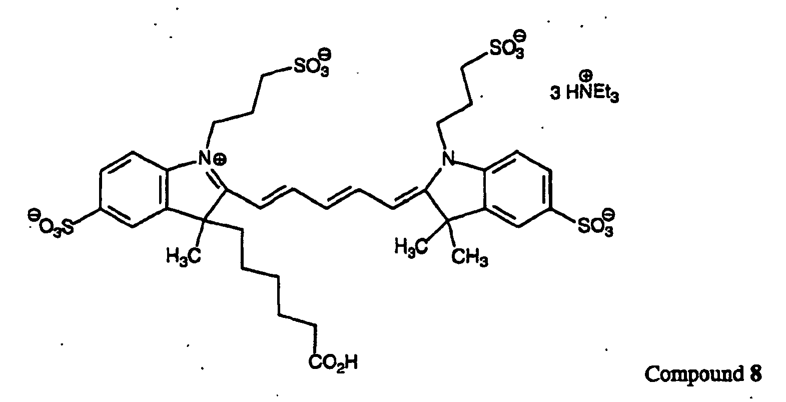

Compound 2 (1 g) and Compound 4 (1.5 g) are combined with 0.84 mL triethylamine and 0.5 mL acetic anhydride. The mixture is stirred at room temperature for 1 hour, then evaporated and the residue is purified by HPLC.

### Example 9. Preparation of Compound 9. (Reference)

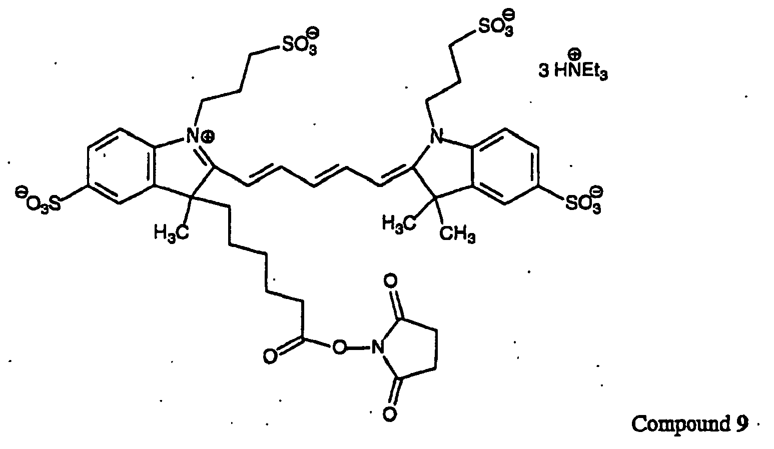

To 55 mg of Compound 8 in 1 mL of DMF is added 0.034 mL of triethylamine and 21 mg of 2-succnimido-1,1,3,3-tetramethyluronium tetrafluoroborate. The mixture is stirred at room temperature for 30 minutes and evaporated to yield the succinimidyl ester.

### Example 10. Preparation of Compound 10. (Reference)

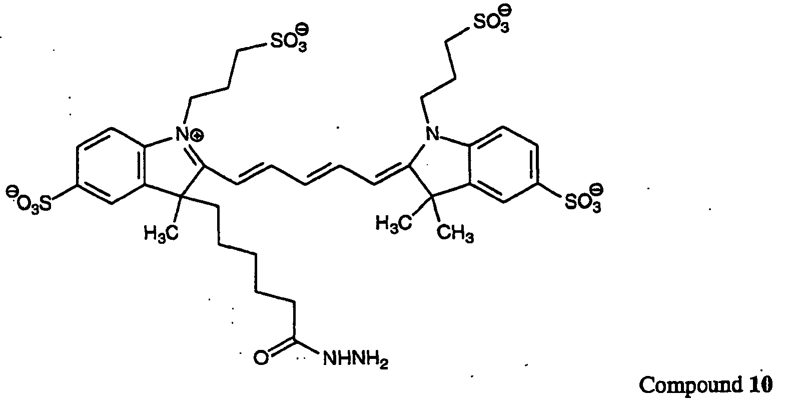

To Compound **9** in acetonitrile is added 3 equivalents of triethylamine and 1.2 equivalents anhydrous hydrazine. The mixture is stirred at ambient temperature for 15 minutes. The product is precipitated with 4 volumes of ethyl acetate and purified by HPLC.

### Example 11. Preparation of Compound 11. (Reference)

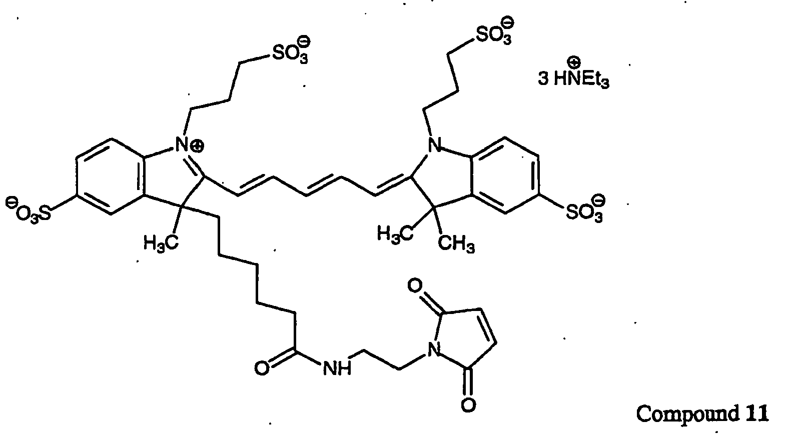

To Compound 9 in acetonitrile at room temperature is added 4 equivalents of triethylamine and 1.2 equivalents of N-(2-aminoethyl)maleimide, trifluoroacetic acid salt. The mixture is stirred at ambient temperature for 15 minutes. The product is precipitated with 4 volumes of ethyl acetate and purified by HPLC.

### Example 12. Preparation of Compound 12. (Reference)

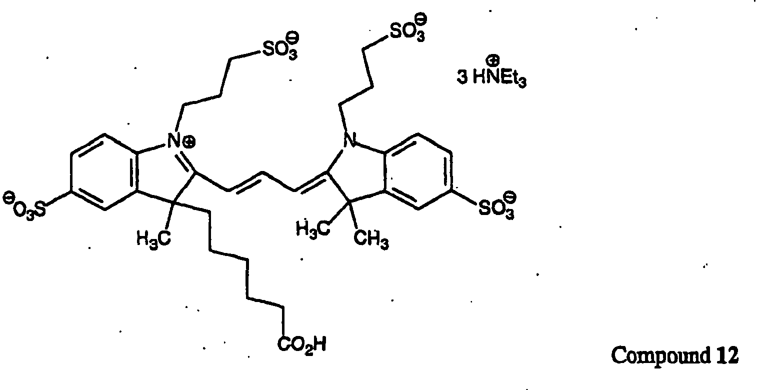

To 6 mmole of Compound 2 is added 2 g of Compound 5 in 20 mL DMF, 4.2 mL triethylamine, and 1.8 mL of acetic anhydride. The reaction is stirred at room temperature for one hour, then evaporated and the residue is purified by HPLC.

### Example 13. Preparation of Compound 13. (Reference)

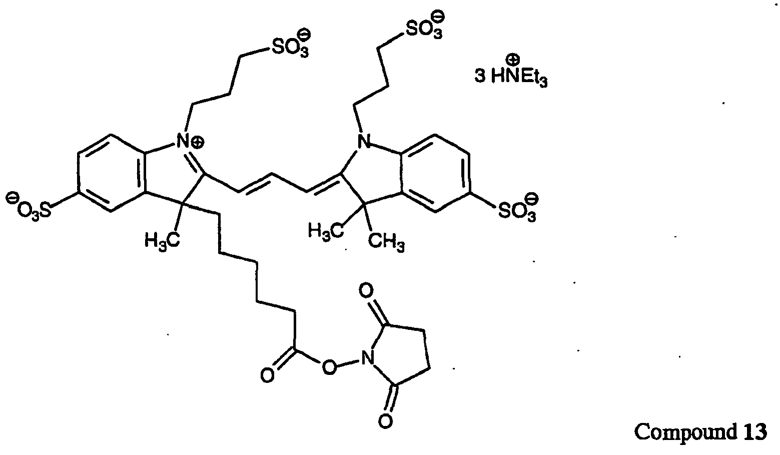

The procedure is similar to that used to prepare Compound **9,** using Compound **12** in place of Compound **8**.

### Example 14. Preparation of Compound 14. (Reference)

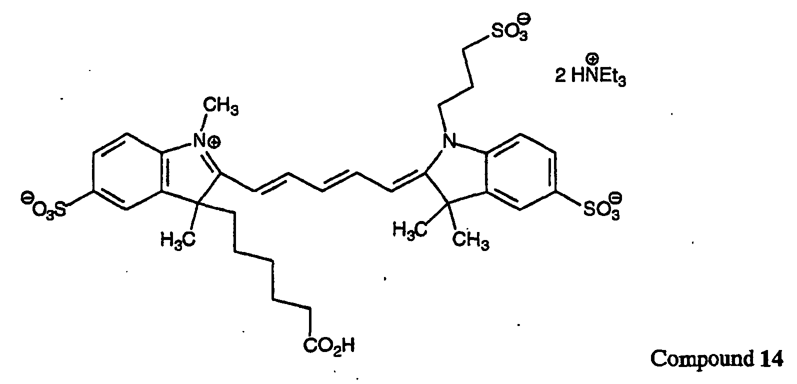

The compound is prepared in DMF by mixing one equivalent each of Compound **4** and Compound **7**, followed by addition of four equivalents of triethylamine and 1.5 equivalents of acetic anhydride. After stirring at room temperature for 2 hours and evaporation, the residue is purified by HPLC.

### Example 15. Preparation of Compound 15.

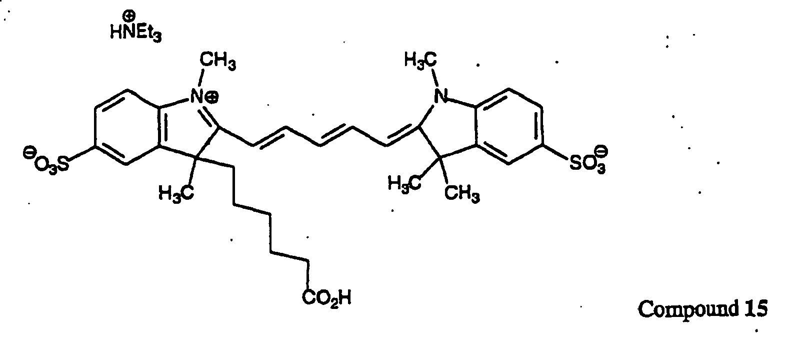

To a mixture of 0.27 g Compound 6 and 0.6 mmoles Compound 7 in 8 mL of DMF is added 0.42 mL triethylamine and 0.1 mL acetic anhydride. The mixture is stirred at room temperature for two hours then evaporated and the residue is purified by HPLC.

### Example 16. Preparation of Compound 16. (Reference)

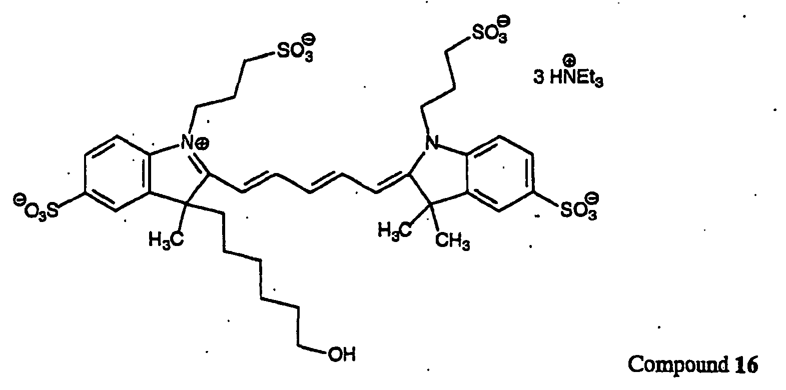

Ethyl 2-methylacetoacetate is alkylated with 6-benzoyloxy-1-bromiohexane in the presence of 1.2 equivalents of sodium hydride in THF and the resulting product was hydrolyzed and decarboxylated in aqueous NaOH as in example 1 to generate the desired 9-hydroxy-3-methyl-2-nonanone. The nonanone is then heated at reflux with 1 equivalent of 4-hydrazinebenzenesulfonic acid in acetic acid to generate 3-(6-hydroxyhexyl)-2,3-dimethyl-5-sulfoindolium, inner salt. The hydroxy again is protected as an benzoyloxy group and this intermediate is then transformed to the protected form of target compound as in example 8. The benzoyl protecting group is then removed by dilute NaOH.

### Example 17. Preparation of Compound 17. (Reference)

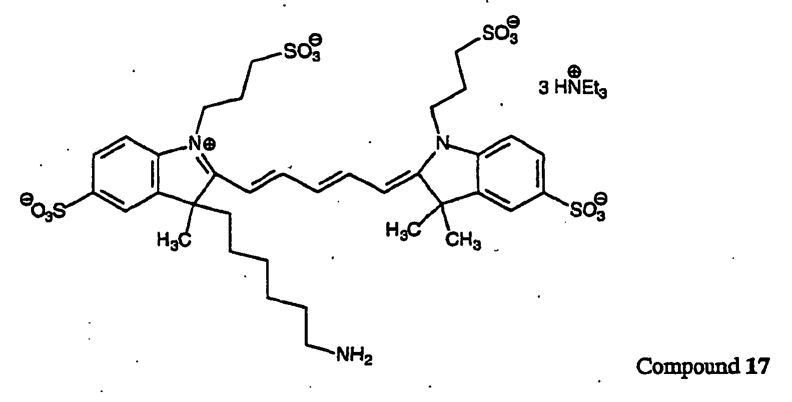

The intermediate is prepared as in Example 16, except that 6-t-butoxycarbonyloxy-1-bromhexane is used instead of 6-benzoyloxy-1-bromohexane. The t-BOC protecting group is removed with trifluoroacetic acid at room temperature after formation of the target dye.

### Example 18. Preparation of Compound 18. (Reference)

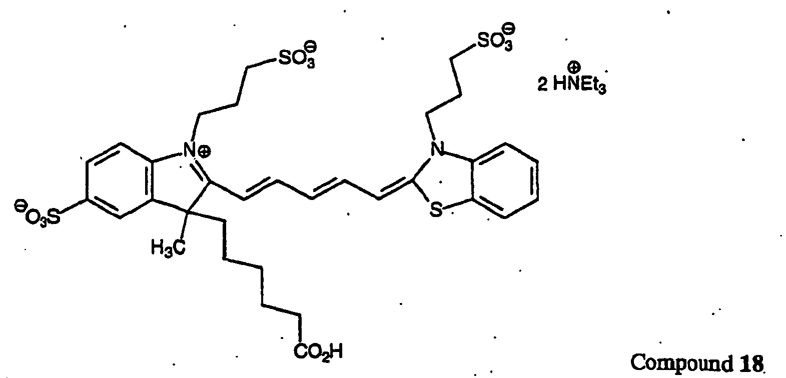

To 1, mmole 2-methyl-1-(3-sulfopropyl)-benzothiazolium, inner salt (from heating of one equivalent each of propanesultone and 2-methybenzothiazole at 110°C for one hour) and 150 mg of Compound 31 in 5 mL of DMF is added 0.28 mL, of triethylamine and 0.1 mL acetic anhydride. The reaction is stirred at room temperature for one hour, then evaporated and the residue is purified by HPLC.

### Example 19. Preparation of Compound 19. (Reference)

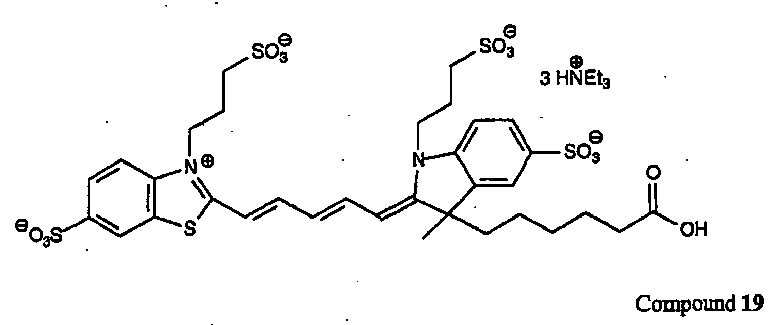

Compound 19 is prepared in the same manner as Compound 18 except starting with 2-methyl-6-sulfobenzothiazole, which is prepared by reaction of sulfuric acid and 2-methylbenbenzothiazole at room temperature.

### Example 20. Synthesis of 2-(6-anilinohexatirenyl)-3,3-dimethyl-5-sulfo-1-(3,3-dimethyl-5-sulfo-1-(3-sulfopropylindolium), inner salt (Compound 20). (Reference)

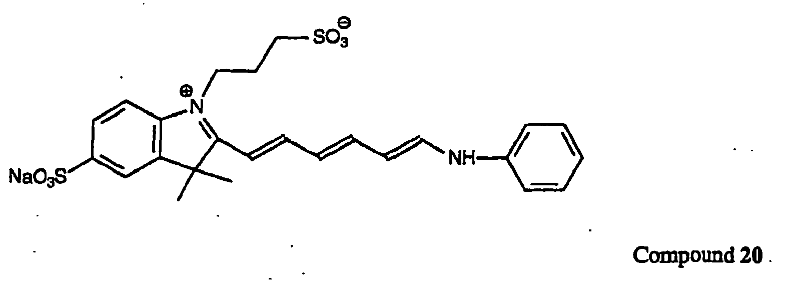

A mixture of 1.9 g of Compound 3A and 2.85 g of N-(5-anilino-2,4-pentadienylidene)aniline hydrochloride in 30 mL of acetic anhydride is heated at 120°C for 30 minutes, At the end of the period, 90 mL of ethyl acetate is added and the product is filtered and used as is.

### Example 21. Synthesis of Compound 21. (Reference)

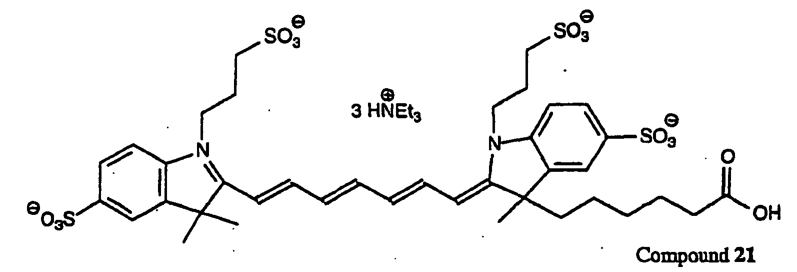

A mixture of 1.05 g of Compound 20, 2 mmoles of Compound 2,10 mL of DMF, 1.7 mL of triethylamine and 0.6 mL of acetic anhydride is stirred at room temperature overnight and then at 35°C for an additional 1.5 hour. 40 mL of ethyl acetate is added and the precipitate is purified by HPLC.

### Example 22. Synthesis of Compound 22. (Reference)

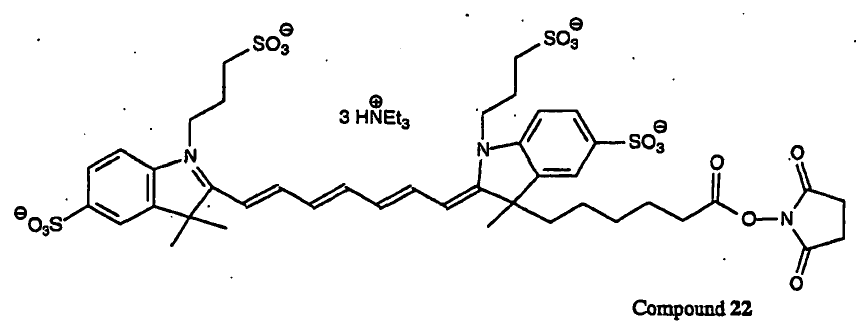

The succinimidyl ester of Compound 21 (Compound 22) is prepared as described in Example 9.

### Example 23. Synthesis of Compound 23. (Reference)

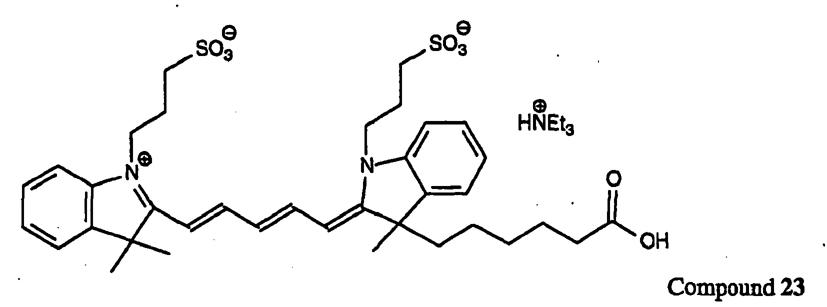

A mixture of 037 g of 2-(4-anilinobutadienyl)-3,3-dimethyl-l-(3-sulfopropyl)indolium. (prepared by the reaction of trimethylindoline and propanesultone as in Example 3 followed by reaction with malonaldehyde dianil hydrochloride as in Example 4), 1.35 mmoles of 3-(5-carboxypentyl)-3-methyl-1-(3-sulfopropyl)indolium (prepared by the reaction of 7-metyl-8-oxononanoic acid and phenyl hydrazine, as in Example 1), 7 mL DMF, 0.42 mL triethylamine and 0.1 mL of acetic anhydride is stirred at room temperature for one hour. Ethyl acetate (30 mL) is added and the precipitate is purified on silica gel to yield 55 mg of Compound 23.

### Example 24. Synthesis of Corresponding Activated Esters from Free Acids.

The following activated esters are prepared from the corresponding free acids, according to the method in Example 9:
Compound 24, prepared from Compound 15 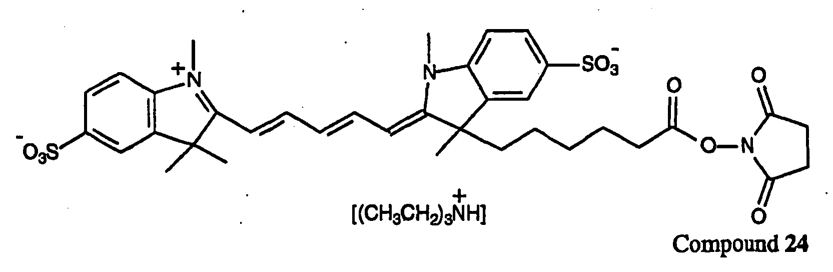
Compound **25,** prepared from Compound 23 (Reference)

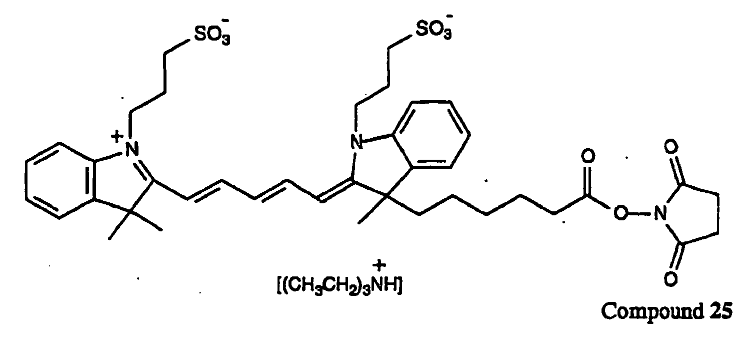

### Example 25. Preparation of Compound 28. (Reference)

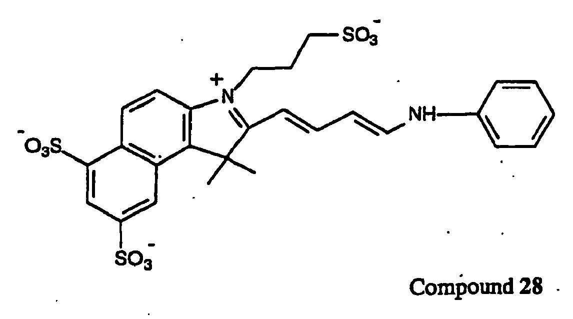

The compound is prepared by quarternization of 1,1,2 trimethylbenzindoleninium 1,3-disulfonate (Bioconjugate Chem., 356-362 (1996)) with propanesultone and then heated with 2 equivalents of malonaldehyde dianil hydrochloride in acetic acid with catalytic amount of triethylamine to yield Compound 28.

### Example 26. Preparation of Compound 29. (Reference)

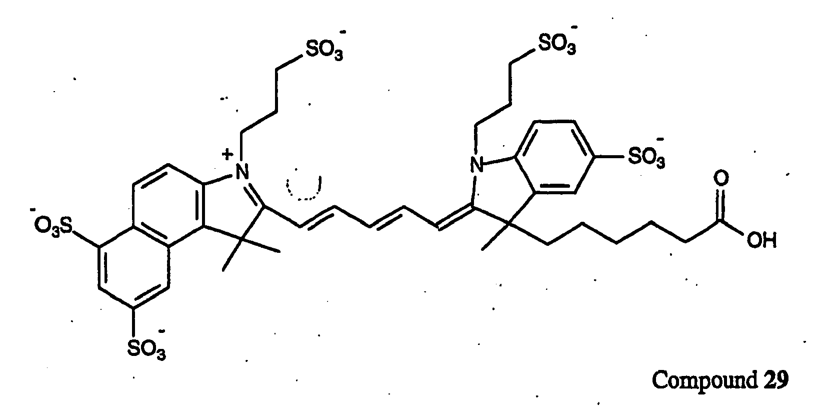

The compound is prepared by stirring one equivalent each of Compound 28 and 3-(5-carboxypentyl)-2,3-dimethyl-5-sulfo-1-(3-sulfopropyl)-indoleninium inner salt, sodium salt in the presence of 3 equivalents of triethylamine and one equivalent of acetic anhydride in DMF at room temperature for one hour to yield Compound 29. Compound 29 is optionally converted to its corresponding succinimidyl ester as described in Examples 9 and 24.

### Example 27. Preparation of Compound 30:

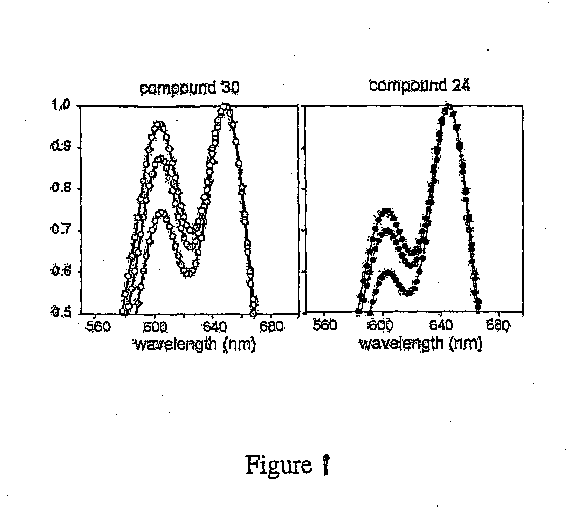

For purposes of comparison with dyes of the invention (See Figure 1), Compound **30** is prepared according to BIOCONJUGATE CHEM. 4, 105-111 (1993).

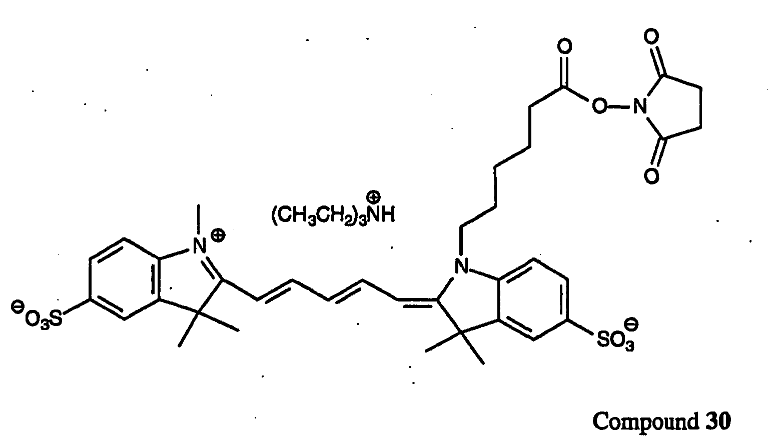

### Example 28. Preparation of Compound 31. (Reference)

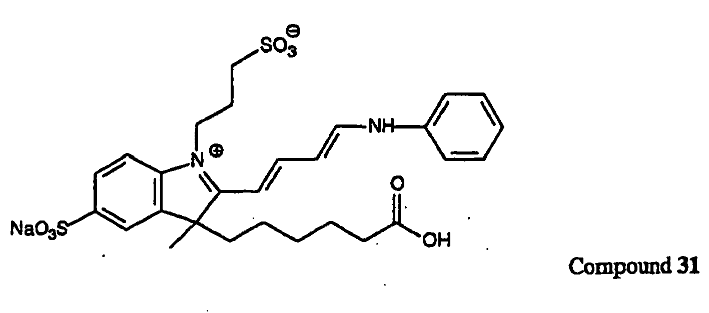

A mixture of 45 mmoles of Compound 2 and 23 g of malonaldehyde dianil hydrochloride is heated to reflux in 400 mL of acetic acid with 0.65 mL of triethylamine for 1 hour. The solvent is evaporated and the residue is purified on silica gel to yield 2.4 g of the product.

### Example 29. Preparation of 7-(3-sulfopropyl)-2,3,3-trimethyl-3H-pyrrolo[2,3-b] pyridinium inner salt (Compound 51). (Reference)

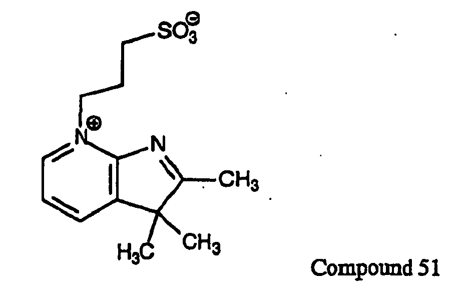

A mixture of 9 g of 2,3,3-trimethyl-3H-pyrrolo[2,3-b]pyridine and 20.6 g of propanesultone is heated at 60 °C for 3 hours. The reaction mixture is then dissolved in 100 mL of acetonitrile and 300 mL of ethyl acetate is added. The resulting sticky solid is again stirred in 300 mL of ethyl acetate to yield 22 g of the product.

### Example 30. Preparation of 5-bromo-2,3,3-trimethyl-3H-pyrrolo[2,3-b]pyridine (Compound 52). (Reference)

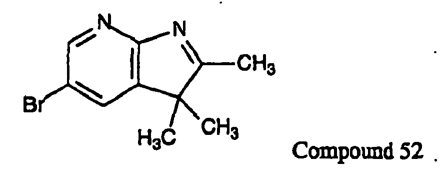

To 40 g of 2,5-dibromopyridine in 200 mL of 2-methoxyethanol is added 53 mL of anhydrous hydrazine and the mixture is heated at 110 °C for 3 hours to generate the 5-bromo-2-hydroazinopyridine. A mixture of 10 g of this hydrazinopyridine is heated at reflux overnight with 11 mL of 3-methyl-2-butanone in 40 mL of benzene equipped, using a condenser equipped with a Dean Stark trap. All of the volatile components are removed under reduced pressure and the resulting residue is heated in 62 g of polyphosphoric acid at 140 °C for 45 minutes. The reaction mixture is poured into water, neutralized with sodium hydroxide and extracted with ethyl acetate. The resulting crude residue is purified by chromatography on silica gel, eluting with 1:1 ethyl acetate/hexanes, to yield 1.44 g of the product.

### Example 31. Preparation of 5-bromo-7-(3-sulfopropyl)-2,3,3-trimethyl-3H-pyrrolo[2,3-b]pyridinium, inner salt (Compound 53). (Reference)

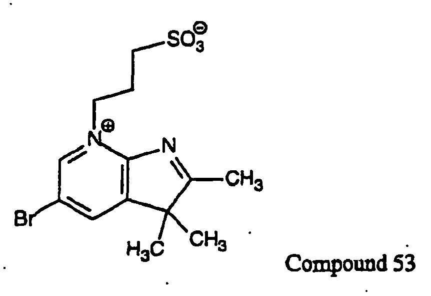

A mixture of 1 g of 5-bromo-2,3,3-trimethyl-3H-pyrrolo[2,3-b]pyridine (Compound 52, Example 30) and 1.54 g of propanesultone is heated at 65 °C for 2 hours. Ethyl acetate is added and the resulting mixture is stirred at room temperature overnight to yield 2.26 g of the product.

### Example 32. Preparation of Compounds 54 and 55. (Reference)

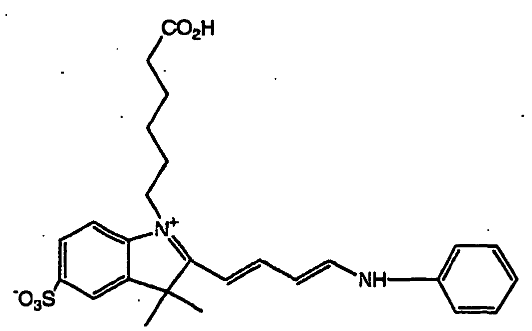

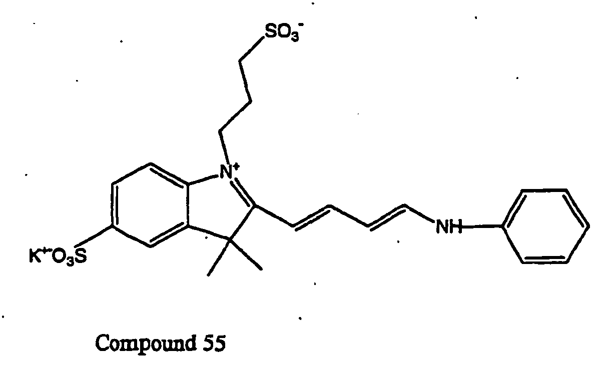

2-(4-Anilinobutadienyl)-1-(5-carboxypentyl)-3,3-dimethyl-5-sulfoindolinium, inner salt and 2-(4-anilinobutadienyl)-1-(3-sulfopropyl)-3,3-dimethyl-5-sulfoindolnium, potassium salt (Compounds 54 and 55, respectively) are prepared according to a literature procedure (Mujumdar, et al, BIOCONJUGATE CHEMISTRY 4, 105-111 (1993))

### Example 33. Preparation of succinimidyl esters of carboxylic acids.

Succininimidyl ester derivatives are typically prepared from the corresponding carboxylic acids using the 2-succinimido-1,1,3,3-tetramethyl uronium tetrafluoroborate (Bannwarth et al. TETRAHEDRON LETT. 1157-1160 (1991)) and either triethylamine or diisopropylethylamine. Succinimidyl ester derivatives are also readily prepared by coupling a carboxylic acid derivative to N-hydroxysuccinimide using an activating agent such as a carbodiimide.

### Example 34. Preparation of 7-(carboxypentyl)-2,3,3-trimethyl-3H-pyrrolo[2,3-b] pyridinium bromide (Compound 59). (Reference)

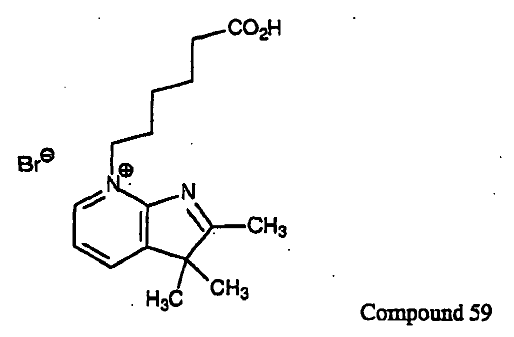

A mixture of 0.54 g of 2,3,3-trimethyl-3H-pyrrolo[2,3-b]pyridine and 1.32 g of 6-bromohexanoic acid is heated at 120 °C for one hour. Ethyl acetate (10 mL) is added, and the reaction mixture is heated at reflux for 15 minutes, then cooled to room temperature. The supernatant liquid is decanted to yield the product.

### Example 35. Preparation of Compound 66 and Compound 67. (Reference)

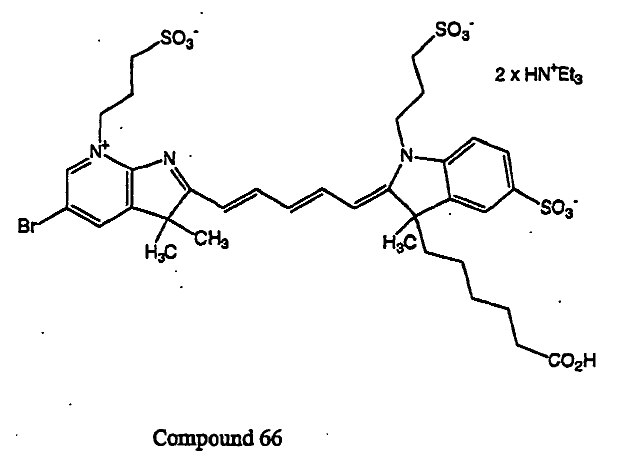

A mixture of 032 g of 2-(4-anilinobutadienyl)-3-carboxypentyl-3-methyl-5-sulfo-1-sulfopropylindolinium, sodium salt (Compound 31, Example 28), 0.75 g of 5-bromo-7-(3-sulfopropyl)-2,3,3-trimethyl-3H-pyrrolo[2,3-b]pyridinium, inner salt (Compound 53, Example 10), 0.36 mL of triethylamine and 0.1 mL of acetic anhydride is stirred in 13 mL of DMF at room temperature for 1 h. 50 mL of ethyl acetate is added then the crude solid is filtered and purified by HPLC. A succinimidyl ester derivative (Compound 67) is prepared according to the methods provided in Example 33.

### Example 36. Preparation of Compound 68 and Compound 69. (Reference)

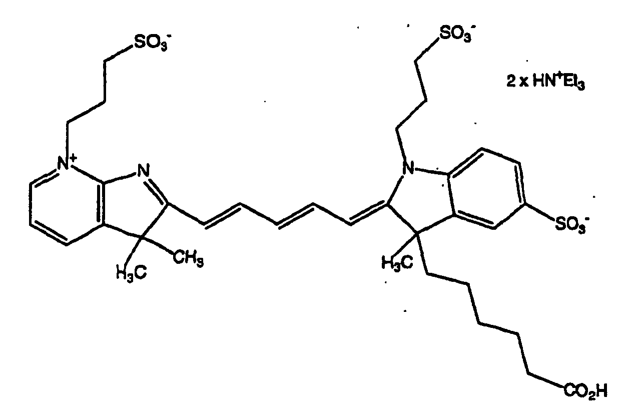

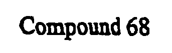

The compound is prepared in a similar manner as Compound 66 except that 7-(3-sulfopropyl)-2,3,3-trimethyl-3H-pyrrolo[2,3-b]pyridinium, inner salt (Compound 51, Example 8) is used. The crude product is purified by HPLC. A succinimidyl ester derivative (Compound 69) is prepared according to the methods provided in Example 33.

### Example 37. Preparation of Compound 72. (Reference)

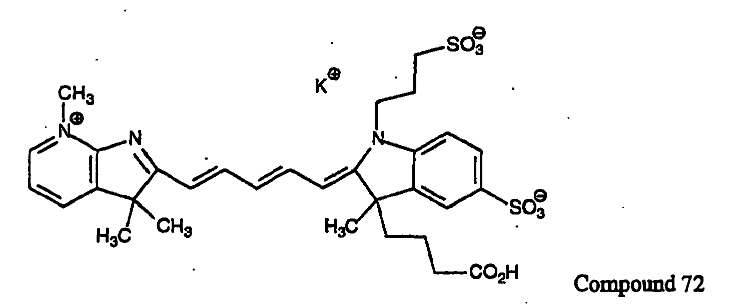

A mixture of 150 mg of 2-(anilimobutadienyl)-3-(3-carboxypropyl)-5-sulfo-1-sulfopropylindolenium, inner salt, potassium salt, 160 mg of 2,3,3,7-tetramethyl-3H-pyrrolo[2,3-b]pyridinium tosylate, 2 mL of DMF, 0.13 mL of triethylamine and 0.08 mL of acetic anhydride is heated at 40 °C for 30 minutes. Volatile components are evaporated and the crude residue is purified on a silica gel column.

### Example 38. Preparation of Compound 73 and Compound 74. (Reference)

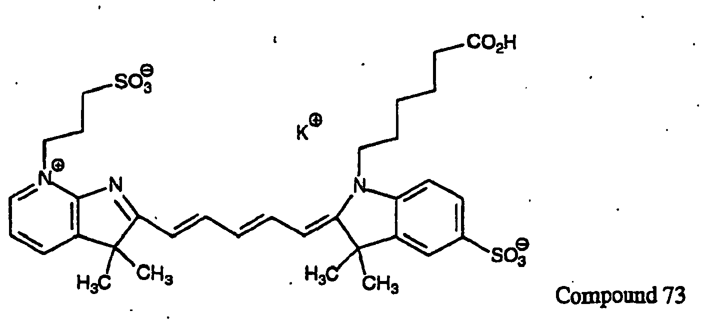

A solution of 3,2 g of 7-(3-sulfopropyl)-2,3,3-trimethyl-3H-pyxrolo[2,3-b]pyridinium, inner, salt and 4 g of 2-(4-anilmobutadienyl)-1-(5-carboxypentyl)-3,3-dimethyl-5-sulfoindolinium, inner salt in 40 mL of DMF, 3.4 mL of triethylamine and 2 mL of acetic anhydride is stirred at room temperature for 4 hours. At the end of the period, 700 mL of acetonitrile is added and the crude solid is recovered by filtration and purified by HPLC. The succinimidyl ester derivative (Compound 74) is generated by the methods provided in Example 33.

### Example 39. Preparation of Compound 79. (Reference)

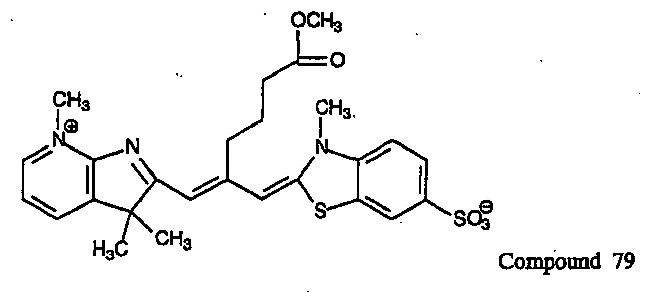

To 1.7 g of 2,3-dimethyl-6-sulfobenzothiazolium tosylate in 20 mL of pyridine at room temperature is added 1.03 mL of methyl 5-chloro-5-oxovalezate. The mixture is heated at 50-60 °C for 3 hours. The pyridine solvent is removed under reduced pressure, and the reaction is worked up with chloroform and brine, and purified by silica gel column to yield 0.92 g of 2-(5-methoxycarbonyl-2-oxopentylidene)-3-methyl-3H-benzothiazole. A mixture of 0.45 g of this benzothiazole and 0.45 g of phosphorous oxychloride in 5 mL of dichloroethane is heated at reflux for 2 hours to generate 2-(2-chloro-2-methoxycarbonylpropylvinyl)-3-methylbenzothiazolium chloride. The volatile components are evaporated and the crude chloride is used without further purification. The crude chloride and 0.45 g of 2,3,3,7-tetramethyl-3H-pyridinium tosylate is stirred in 5 mL of dichloroethane in the presence of 0.45 mL of triethylamine for 2 hours. The volatile components are removed under reduced pressure, and the residue is dissolved in 5 mL of methanol and added dropwise to a solution of 4.5 g of sodium iodide in 30 mL water. The sticky solid is purified on a silica gel column to yield Compound 79.

### Example 40. Preparation of Compound 82 and Compound 86. (Reference)

6-Hydrazinonaphthalene 1,3-disulfonate (Bioconj. Chem., 356-362(1996)) is heated with 7-methyl-8-oxononanoic acid in acetic acid to generate the 1-carboxypentyl-1,2-dimethyl-6,8-disulfobenzindolenine. The benzindolenine is quarternized with propane sultone and chain elongated with malonaldehyde dianil hydrochloride to yield the 2-(4-anilinobutadienyl) derivative (Compound 82) which is then reacted with Compound 53 (Example 31) in the presence of acetic anhydride and triethylamine to yield the desired product.

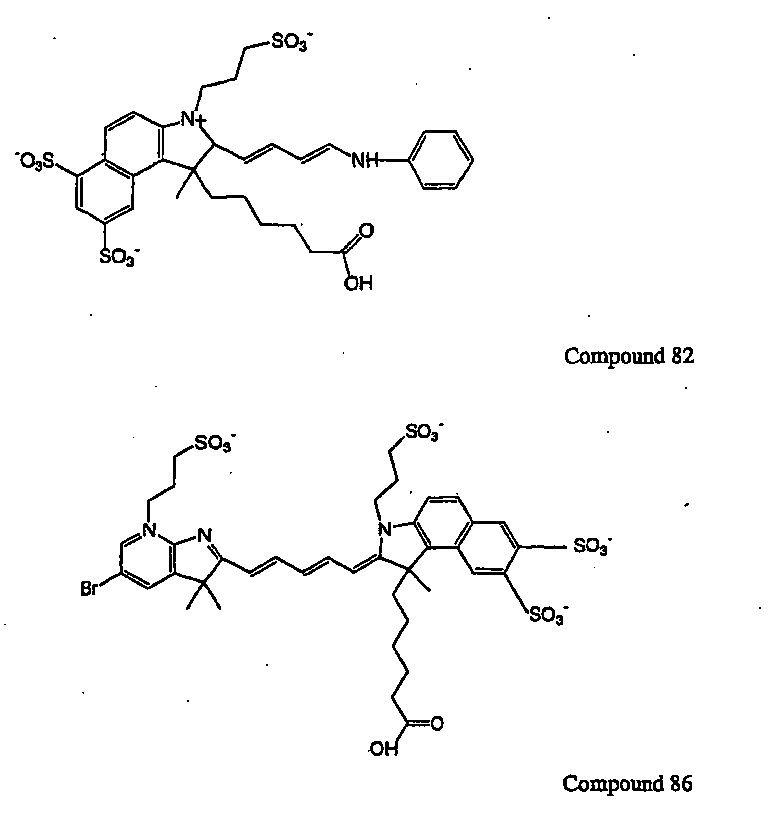

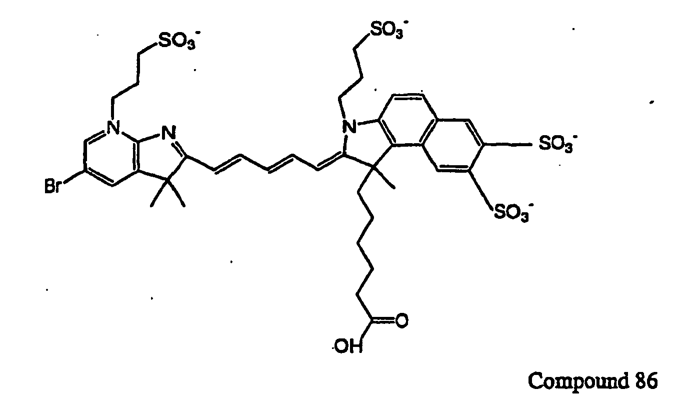

### Example 41. Preparation of Compound 83. (Reference)

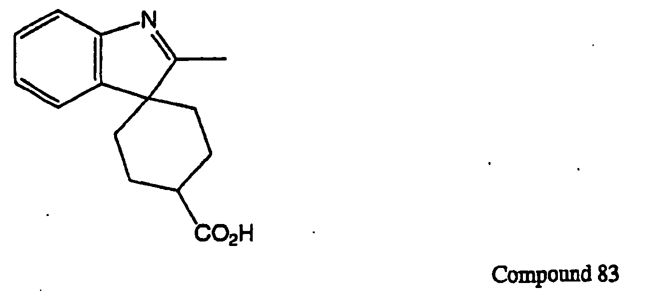

A mixture of 100 mg of 4-acetylcyclohexanecarboxylic acid and 120 mg of hydrazinobenzenesulfonic acid is refluxed in 5 mL of acetic acid to obtain the desired compound.

### Example 42. Preparation of Compound 84. (Reference)

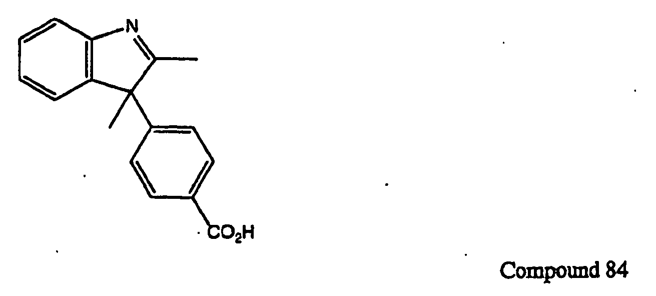

A mixture of 2-(4-carboxyphenyl)-butan-2-one and hydrazinobenzenesulfonic acid in acetic acid is refluxed for 3 hours to obtain the desired product.

### Example 43. Preparation of Compound 85. (Reference)

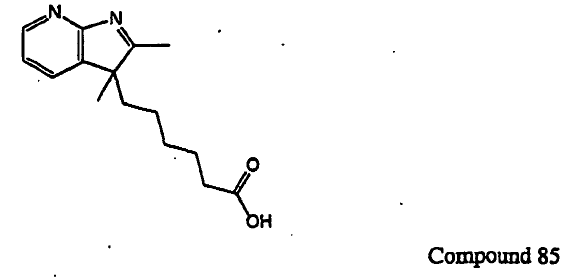

A mixture of 0.85 g of 2-hydrazinopyridine and 1.6 g of 7-methyl-8-oxo-nonanoic acid is refluxed in 10 mL of benzene overnight. The volatile components are evaporated and the residue is heated with 0.2 g of zinc chloride at 250° C for one hour to obtain the desired product.

### Example 44. Preparation of protein dye-conjugates.

A series of dye conjugates of goat anti-mouse IgG (GAM), goat anti-rabbit IgG (GAR), streptavidin, transferrin and other proteins, including R-phycoerythrin (R-PE) and allophycocyanin (APC) are prepared by standard means (Haugland et al., METH. MOL. BIOL. 45, 205 (1995); Haugland, METH. MOL. BIOL. 45, 223 (1995); Haugland, METH. MOL. BIOL. 45, 235 (1995); Haugland, CURRENT PROTOCOLS IN CELL BIOLOGY 16.5.1-16.5.22 (2000)) using Compound 9 (Reference dyes) or 74 (Reference dyes) and a mono-succinimidyl. ester derivative of the Cy5 dye (Amersham-Pharmacia Biotech).

The typical method for protein conjugation with succinimidyl esters of the invention is as follows. Variations in ratios of dye to protein, protein concentration, time, temperature, buffer composition and other variables that are well known in the art are possible that still yield useful conjugates. A solution of the protein is prepared at ~10 mg/mL in 0.1 M sodium bicarbonate. The labeling reagents are dissolved in a suitable solvent such as DMF at -10 mg/mL. Water is a suitable solvent for many dyes of the invention. Predetermined amounts of the labeling reagents are added to the protein solutions with stirring. A molar ratio of 10 equivalents of dye to 1 equivalent of protein is typical, though the optimal amount varies with the particular labeling reagent, the protein being labeled and the protein's concentration, and is determined empirically. When optimizing the fluorescence yield and determining the effect of degree of substitution (DOS) on this brightness, it is typical to vary the ratio of reactive dye to protein over a several-fold range. The reaction mixture is incubated at room temperature for one hour or on ice for several hours. The dye-protein conjugate is typically separated from free unreacted reagent by size-exclusion chromatography, such as on BIO-RAD P-30 resin equilibrated with phosphate-buffered saline (PBS), The initial, protein-containing. colored band is collected and the degree of substitution is determined from the absorbance at the absorbance maximum of each fluorophore, using the extinction coefficient of the free fluorophore. The dye-protein conjugate thus obtained can be subfractionated to yield conjugates with higher, lower or more uniform DOS.

A solution of the desired protein is prepared at 10 mg/mL in 0.1 M sodium bicarbonate. The labeling reagents are dissolved in DMF at 10 mg/mL. Predetermined amounts of the labeling reagents are added to the protein solutions with stirring. A molar ratio of 10 equivalents of dye to 1 equivalent of protein is typical, though the optimal amount varies with the particular labeling reagent, the protein being labeled and the protein's concentration, and is determined empirically. The reaction mixture is incubated at room temperature for one hour, or on ice for several hours. The dye-protein conjugate is typically separated from free unreacted reagent by size-exclusion chromatography on BIO-RAD P-30 resin, equilibrated with PBS. The initial, protein-containing colored band is collected and the degree of substitution is determined from the absorbance at the absorbance maximum of each fluorophore, using the extinction coefficient of the free fluorophore.

**Table 5: Fluorescence of Protein Conjugates of Compound 69 (Example 36) (Reference)**

| Protein | DOS* | Quantum Yield† |
|---|---|---|
| Goat anti-Mouse IgG | 3.7 | 0.47 |
| Streptavidin | 4.5 | 0.85 |
| Wheat Germ Agglutinin | 3.1 | 0.32 |
| Goat anti-Rabbit IgG (highly cross-absorbed) | 4.4 | 0.5 |
| Goat anti-Chicken IgG | 4.5 | 0.33 |
| Rabbit anti-Mouse IgG | 3.0 | 0.67 |
| Goat anti-Mouse IgG (highly cross-absorbed | 4.4 | 0.63 |
| Goat anti-Guinea Pig IgG | 5.1 | 0.33 |
| Protein A(MR=4)‡ | 2.1 | 0.64 |
| Protein A (MR=8)‡ | 4.9 | 0.39 |

| | | |
|---|---|---|
| *Extinction coefficients are determined for the free carboxylic acid in aqueous solution †Quantum yield measured relative to DDAO (7-hydroxy-9H-(1,3-dichloro-9,9-dimethylacridine-2-one) ‡MR is the approximate molar ratio of the dye to protein following conjugation | | |

**Table 6: Fluorescence of Protein Conjugates of Compound 67 (Example 35) (Reference)**

| Protein | DOS* | Quantum Yield |
|---|---|---|
| Goat anti-Mouse IgG | 5.9 | 0.15 |
| Streptavidin | 4.8 | 0.66 |
| Goat anti-Rabbit IgG (highly cross-absorbed) | 4.9 | 0.26 |
| Goat anti-Chicken IgG | --- | --- |
| Rabbit anti-Mouse IgG | 6.0 | 0.31 |

| | | |
|---|---|---|
| *Extinction coefficients are determined for the free carboxylic acid in aqueous solution | | |

Protein conjugates of antibody fragments, of other avidins and of other proteins are prepared and analyzed similarly.

### Example 45. Total fluorescence of selected dye-proteing conjugates as a function of degree of substitution (DOS).

The conjugates of Compound 9 (Reference dye) exhibit equal or greater fluorescence than the conjugates of Cy5 dye at similar DOS when conjugated to a wide variety of proteins (Table 3), Protein conjugates are prepared (as described in Example 44) at varying DOS and compared for brightness (total-fluorescence, TF) and relative quantum yield (RQY; definition at bottom of Table 3). Total fluorescence is proportional to the overall brightness of the bioconjugate, and is defined as the product of the RQY and DOS: TF= RQY X DOS.

### Example 46. Total fluorescence of selected dye-protein conjugates as a function of degree of substitution.

Table 3 shows the heavy quenching of the fluorescence of Cy5 conjugates (at even moderate DOS). Comparing GAM IgG Compound 9 (Reference dye) at - DOS 4.2 with a GAM IgG Cy5 at DOS - 4.8 (see Table 3), reveals that the Compound 9 bioconjugate is approximately 5.0/0.4 brighter (12.5X) than the Cy5 bioconjugate. This type of pattern is observed for all of the proteins in Table 3. In general, the higher the DOS, the brighter Compound 9 bioconjugates are relative to the Cy5 bioconjugates, although, as can be seen, the Compound 9 bioconjugates are brighter at all DOS tested.

The decrease in the RQY of the Cy5 bioconjugates is found to be accompanied by an increase in the 600-nm absorbance band relative to the 650-nm absorbance band. This effect is true for all of the bioconjugates listed in Table 3. The increase in extinction of the 600 nm band is always associated with a large quenching of the fluorescence. Gruber et. al. (supra) who observed a similar correlation of an increased absorbance at 600 nm and a large decrease in fluorescence intensity. This general observation has now been confirmed with several other proteins (Table 3).

The Cy5 and Compound 9 derivatives of GAM are examined using both absorbance spectroscopy and fluorescence excitation spectroscopy. The fluorescence excitation spectra (emission wavelength = 725 nm) of each derivative is normalized to its absorbance spectra at 660 nm. The Cy5 GAM 600 nm absorbance band does not emit fluorescence, as evidenced by the large difference between the excitation and absorbance in this region of the spectrum. The absorbance and excitation spectra of Compound 9 bioconjugates nearly overlap in this region and hence show a drastically reduced amount of fluorescence quenching.. When this same Cy5-GAM antibody is dissolved in 6.0 M guanidinium hydrochloride (pH= 7.5), the 600 nm absorbance band greatly decreases, the 650 nm absorbance band increases, and the overall fluorescence intensity increases dramatically. This result indicates that it is the behavior of the Cy5 derivative on the bioconjugate native structure, that causes the large decrease in fluorescence. A similar result was obtained for Cy5 derivatives of nucleate-digested DNA (Example 59).

### Example 47. Comparison of two structural "Cy5-like" isomers (Compound 30 and Compound 24).

In order to better understand the origin of the anomalous absorption effects of Cy5 bioconjugates and the much smaller effect with Compound 9 (Reference dye), two isomers of Cy5 were synthesized and conjugated to GAR at various DOS (Compound **30** and Compound **24**). Figure 4 is a direct comparison of absorbance properties of these two isomers at DOS's of approximately 2.8, 4.3, and 5.5 on GAR. The only difference between the chemical structures of Compounds 30 and 24 is the change in position of the reactive moiety from position 1 (Cy5 position) to position 3 (shared by all dyes of the invention) of the indolium ring. One can see that this chemical change has resulted in a drastic improvement of the behavior of the absorbance of Compound 24 over Compound 30. Compound 24 is also a brighter fluorescence emission than Compound 30 at all of these tested DOS's (Table 3).

### Example 48. Comparison of the total fluorescence of GAM conjugates

Conjugates of the Cy5 dye with GAM were purchased from several sources (Table 3). Absorption spectra of each of these conjugates confirm that the Cy5 GAM conjugates all had much larger absorbances at 600 nm (relative to the 650 nm band) than the corresponding Compound 9-GAM conjugates (Reference dye). For fluorescence brightness comparisons, the proteins are adjusted to approximately the same concentration as measured by the absorption at 280 nm corrected for the contribution of dye absorption at 280 nm. For some commercial Cy5 bioconjugates, the protein concentration supplied by the vendor was utilized due to the presence of 280 nm absorbing stabilizers. The conjugates are excited at 633 nm and the fluorescence emission spectrum measured. The results in Table 3 confirm the superior fluorescence brightness (TF) of GAM conjugates of Compound 9 compared with the commercially available Cy5 conjugates (Reference conjugate) of GAM.

The increased brightness is also apparent in experiments performed on a flow cytometer. The flow cytometer intensity comparisons do not rely on the same type of RQY, DOS, and TF calculations required for the spectroscopically based fluorescence intensity comparisons.

Human peripheral blood is drawn in a sodium heparin tube, One hundred µL of blood is added to a Falcon tube. The blood is blocked with 5 µL of mouse monoclonal antibodies to both human CD16 and CD32 (Caltag) for 15 minutes at room temperature. The cells are washed with PBS and resuspended to 100 µL. The blood is then incubated with mouse monoclonal antibody to mouse anti-CD3 (Caltag) at the recommended concentration of 0.50 µg for 30 minutes at 37 degrees Celsius. After incubation with the primary antibody, the cells are washed and resuspended. The blood is then incubated with GAM conjugates of Compound 9 (prepared as in Example 44) and the commercial GAM conjugates of Cy5 from Jackson Laboratories (DOS - 1.9) and Amersham-Phannacia (DOS -11) at a concentration of 0.50 µg for 30 minutes at 37 degrees Celsius. The red blood cells are lysed with a cell lysis buffer and centrifuged to remove the lysed red blood cells. The cell pellet is washed once with PBS and resuspended to a final volume of 500 µL. The samples are analyzed on a FACScan flow cytometer (BD Biosciences) exciting with a 488 nm argon-ion laser and a long-pass (>650 nm) filter. The geometric mean of the background subtracted fluorescence intensity for the Compound 9 conjugates of GAM is 164, whereas the Cy5-GAM conjugates prepared by Jackson Laboratories and Amersham-Pharmacia are 71, and 30, respectively.

Flow cytometry studies are performed as a function of DOS for this antibody, and it is found that at all DOS's, the conjugate of Compound 9 with GAM is from 1.4X to 5.9X brighter than the commercially available Cy5 conjugates of GAM (see Table 7).

| **Table 7** | | |
|---|---|---|
| DOS | (Compound 9 GAM) / (Amersham Cy5 GAM)† | (Compound 9 GAM) / (Jackson Cy5 GAM)‡ |
| 1.83 | 4.84 | 1.92 |
| 2.36 | 5.7 | 2.26 |
| 3.94 | 5.8 | 2.26 |
| 4.36 | 5.86 | 2.33 |
| 7.25 | 3.6 | 1.4 |

| | | |
|---|---|---|
| †, ‡, The geometric mean of the background subtracted fluorescence intensities obtained from Compound 9-labeled GAM divided by the intensity of Amersham-Pharmacia Biotech (†) or the Jackson Laboratories (‡) Cy5 version of this same antibody. | | |

### Example 49. GAR conjugates of Compound 13 and those of the spectrally similar CY3 dye. (Reference example)

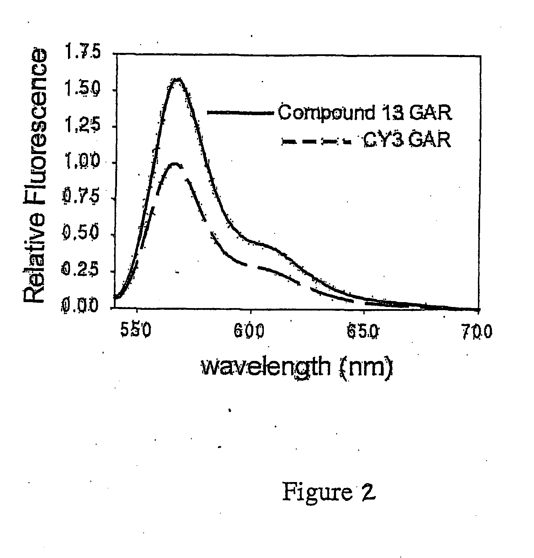

GAR is labeled with Compound 13 and the CY3 reactive dyes at a variety of degrees of substitution ranging from 1.0-12. In all cases, the GAR conjugates of Compound 13 are superior in brightness to the Cy3 dye-labeled GAR (at equivalent DOS). A typical example is shown in Figure 2 (DOS ~6.3). Excitation wavelength = 532 nm.

### Example 50. Photostability comparison.

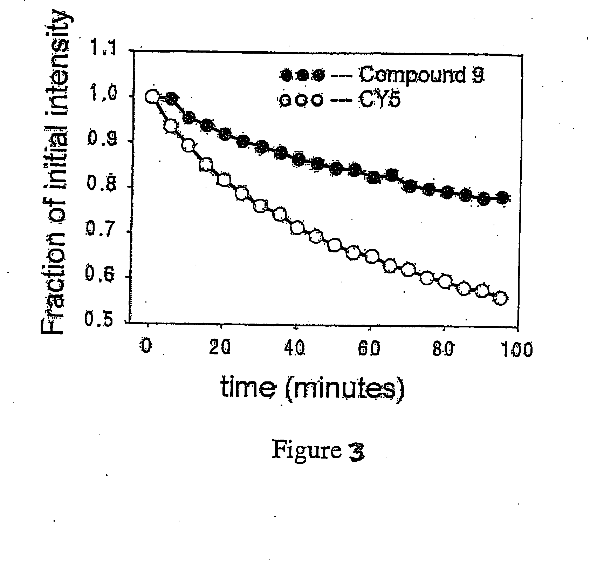

Photobleaching experiments are performed in small capillary tubes at 0.5 µM concentrations of Compound 9 (Reference dye) and commercially available Cy5 reactive succinimidyl esters, in PBS, pH 7.5. The 40X objective of a Nikon Eclipse E-400 and Cy3/Cy5 filter XF92 (Omega) with a 100W Mercury lamp excitation, is utilized. Integrated intensities are collected under constant illumination as a function of time (Figure 3). After 100 minutes of illumination, Compound 9 remains about 2X brighter than the Cy5 dye.

### Example 51. Labeling β-galactosidase with a thiol-reactive dye.

A solution of β-galactosidase, a protein rich in free thiol groups, is prepared in PB S (2.0 mg in 400 µL). The protein solution is then treated with a 20 mg/mL solution of the maleimide derivative Compound 11 (Reference dye) in DMF. Unreacted dye is removed on a spin column. The degree of substitution by the dye is estimated using the extinction coefficient of the free dye. The protein concentration is estimated from the absorbance at 280 nm, corrected for the absorbance of Compound 11 at that wavelength.

### Example 52. Fluorescence energy transfer in conjugates of R-phycoerythrin and Allophycocyanin.

R-phycoerythrin (R-PE) conjugates of Compound 9 (Reference dye) and 74 (Reference dye) are prepared as in Example 44 with a DOS sufficiently high to quench the donor fluorescence almost completely (DOS ~ 4-8). The resulting phycobiliprotein conjugate is excited at 488 nm and the fluorescence emission is compared to that of unmodified R-phycoerythrin excited at the same wavelength. Highly efficient energy transfer (> 99%) occurs from the protein to the fluorescent dye.

Compound **22** (Reference dye) conjugated to R-PE with a DOS of 4.7, 8.2, and 13, generates energy-transfer efficiencies of ~90%, ~99%, and ~99.3%, respectively. A conjugate of these complexes with streptavidin is prepared essentially as described by Haugland (METH. MOL. BIOL. 45, 205 (1995), *supra*). This streptavidin conjugate retains the energy transfer properties and is useful for cell staining in flow cytometers that utilize the argon-ion laser for excitation.

Tandem conjugates of allophycocyanin can also be made, with longer wavelength dyes such as Compound 22 yield emission well beyond 700 nm when excited near 633 nm.

Human peripheral blood is drawn in a sodium heparin tube as in Example 48, except that a biotinylated anti-CD3 antibody (Caltag) is utilized as the primary step, and tandem-dye conjugates of Compound 9-derivatized streptavidin-R-PE and the commercial Cy5 version of this product (Gibco Red 670) are utilized for detection in parallel experiments. The samples are analyzed on a FACScan flow cytometer (BD Biosciences) exciting with a 488 nm argon-ion laser and a long-pass (>650 nm) filter. The signal-to-noise ratio of the tandem conjugate prepared from Compound 9 is - 4.5X brighter than the corresponding Cy5 tandem conjugate.

### Example 53. Preparation of fluorescent-dye labeled microspheres.

Uniform microspheres are conjugated to the dyes of the invention by one of four methods. In Method A, 1.0 µm amme-derivatized polystyrene microspheres are suspended at ~2% solids in 100 mM NaHCO₃, pH 8.3 and treated with 2 mg/mL of an amine-reactive dye. After 1 hour the microspheres are centrifuged and washed with buffer.

In Method B, carboxylate-modified microspheres are suspended in a solution of a protein that has been conjugated to a dye of the invention. The protein is passively adsorbed on the microspheres, and excess protein is removed by centrifugation and washing. Microparticles of a size that cannot be centrifuged are separated from excess protein by dialysis through a semi-permeable membrane with a high MW cutoff or by gel filtration chromatography.

In Method C a dye-labeled protein is covalently coupled through its amine residues to the carboxylate groups of the polymer using ethyl 3-(dimemylainmopropyl)carbodiimide (EDAC).

In Method D, biotinylated microspheres are treated with a streptavidin, avidin or anti-biotin conjugate of a dye of the invention, and the conjugates are isolated as in Method B.

The larger particles can be analyzed for uniformity of staining and brightness using flow cytometry. The microspheres can be further coupled to proteins, oligonucleotides, haptens and other biomolecules for assays using methods well known in the art.

### Example 54. Preparation of fluorescent liposomes using dyes of the invention.

Selected dyes of the invention are sufficiently water soluble to be incorporated into the interior of liposomes by methods well known in the art (J. BIOL. CHEM. 257, 13892 (1982) and PROC. NAIL. ACAD. SCI. USA 75, 4194 (1978)). Alternatively, liposomes containing dyes of the invention having a lipophilic substituent (e.g. alkyl having 11-22 carbons), within their membranes are prepared by co-dissolving the fluorescent lipid and the unlabeled lipids phospholipid(s) that make up the liposome before forming the liposome dispersion essentially as described by Szoka, Jr. et al. (ANN. REV. BIOPHYS. BIOENG. 9, 467 (1980)).

### Example 55. Preparation of nucleotide dye-conjugates.

To 2 mg of 5-(3-aminoanyl)-2'-deoxymidine 5'-triphosphate (Sigma Chemical) in 100 µL water is added Compound 9 (Reference dyes) or 69 (Reference dyes) in 100 µL DMF and 5 µL triethylamine. After 3 hours, the solution is evaporated and the residue is purified by HPLC. The product fractions are lyophilized to give the red-fluorescent nucleotide conjugate.

Alternatively, fluorescent dye-conjugates of deoxyuridine 5-triphosphate are prepared from 5-(3-amino-1-propynyl)-2'-deoxyuridine 5'-triphosphate (as described in Hobbs, Jr. et al, *supra),* or by treating a thiolated nucleotide or a thiophosphate nucleotide with a thiol-reactive dye (such as the maleimide Compound 11).

Additionally, 2'-(or 3')-2-aminoethylaminocarbonyladenosine 5'-triphosphate is reacted with a slight excess of Compound 9 and, following precipitation with ethanol, the ribose-modified product is purified by preparative HPLC.

### Example 56. Preparation of an oligonucleotide dye-conjugate.

A 5'-amine-modified, 18-base M13 primer sequence (~100 µg) is dissolved in 4 µL H₂O. To this is added 250 µg of Compound 9 (Reference dyes) or 69 (Reference dyes) in 100 µL 0.1 M sodium borate, pH 8.5. After 16 hours, 10 µL of 5 M NaCl and 3 volumes of cold ethanol are added. The mixture is cooled to -20°C, centrifuged, the supernatant is decanted, the pellet is rinsed with ethanol and then dissolved in 100 µL H₂O. The labeled oligonucleotide is purified by HPLC on a 300Å C8 reverse-phase column using a ramp gradient of 0.1 M triethylammonium acetate (pH ~7) and acetonitrile (5-95% over 30 min). The desired peak is collected and evaporated to give the fluorescent oligonucleotide.

### Example 57. In situ hybridization of an RNA probe.

Mouse fibroblasts are fixed and prepared for mRNA *in situ* hybridization using standard procedures. A dye-labeled RNA probe is prepared by *in vitro* transcription of a plasmid containing the mouse actin structural gene cloned downstream of a phage T3 RNA polymerase promoter. Labeling reactions comprise combining 2 µL DNA template (1 µg DNA), 1 µL each of 10 mM ATP, CTP and GTP, 0.75 µL 10 mM UTP, 2.5 µL 1 mM aminoallyl-labeled UTP (Example 55), 2 µL 10X transcription buffer (400 mM Tris, pH 8.0, 100 mM MgCl₂, 20 mM spermidine, 100 mM NaCl), 1 µL T3 RNA polymerase (40 units/µL), 1 µL 2 mg/mL BSA, and 8.75 µL water. Reactions are incubated at 37°C for two hours.

The DNA template is removed by treatment with 20 units DNase I for 15 minutes, at 37°C. The RNA transcript is purified by extraction with an equal volume of phenol:chloroform, 1:1, then by chromatography on SEPHADEX G50. Labeled RNA is denatured for 5 minutes at 50°C, then hybridized to cellular preparations using standard procedures. The long-wavelength fluorescence of the labeled cells is detected by excitation through an optical filter optimized for Cy5-like dyes (Omega XF47). The spatially integrated fluorescence from the FISH target region (18 separate intensities) as a function of the number of dyes incorporated per base of probe for conjugates of Compound 9 (Reference dye) and of Cy5 reveal that conjugates of Compound 9 (Reference dye) are brighter than the corresponding Cy5 probe by greater than 50% (at -13 bases per dye incorporation).

### Example 58. Preparing DNA hybridization probes using fluorescent platinum dye (ULS)-Compounds.

A fluorescent platinum complex (ULS) is prepared from dyes of the invention and from Cy5 monosuccinimidyl ester by adapting the methods provided in U.S. Pat. 5,714,327 to Houthoff et al. (1998). For each labeling reaction, a microfuge tube containing 1 µg of pUC1.77 plasmid DNA containing a chromosome 1 human α-satellite probe (DNase treated to a fragment size between 500-1000 bp) in 5 mM Tris, pH 8, 1 mM EDTA, is heated for ~10 minutes at 95°C to fully denature the DNA. The DNA is cooled on ice. 1 µL of a 1 mg/mL solution of the prepared ULS complex is added, followed by the addition of 5 mM Tris, pH 8,1 mM EDTA to bring the total volume to 25 µL. The samples are incubated 15 minutes at 80°C. The reactions are stopped on ice. The labeled DNA is purified on a Bio-Rad Micro Bio-Spin P-30 Tris Chromatography Column. The labeled DNA products are suitable for *in situ* hybridization experiments.

A series of Compound 9 (Reference dye) ULS and Cy5 ULS DNA hybridization probes are examined with the number of dyes per base varying from 0 per 100 bases, to approximately 8 dyes per hundred bases. Similar to the behavior of the Cy5 bioconjugates of proteins (Examples 33, 34) and on aminoallyl-labeled DNA (Example 60), the fluorescently quenched 600 nm absorbance band greatly increases at larger numbers of Cy5 dyes per DNA base (equivalent to higher DOS in protein examples) (open circles, Figure 4), but does not increase with the Compound 9 (Reference dye) ULS-labeled DNA (closed circles, Figure 4).

### Example 59. Preparing DNA hybridization probes using amine-modified DNA and an amine-reactive dye of the invention.

Nick translation is performed using pUC1.77 plasmid DNA containing a chromosome 1 human α-satellite probe. To a microcentrifuge tube is added, in the following order: 23.5 µL H₂O, 5 µL 10X Nick Translation buffer (0.5 M Tris-HCl, 50 mM MgCl₂, 0.5 mg/mL BSA, pH 7.8), 5 µL 0.1 M DTT, 4 µL d(GAC)TP mix (0.5 mM dATP, 0.5 mM dCTP, 0.5 mM dGTP), 1 µL 0.5 mM dTTP, 4 µL 0.5 mM aminoallyl-dUTP, 1 µL 1 µg/µL template DNA, 5 µL DNase I (I µg/mL, 2000 Kunitz units/mg), 1.5 µL DNA polymerase I (10 U/µL). The tube is incubated 2 hours at 15°C, then brought to a final volume of 100 µL with H₂O. The amine-modified DNA is purified using a QIAQUICK PCR purification Kit (Qiagen) with the following modifications to purify the DNA from the enzyme and amine-containing Compounds: 75% EtOH is substituted for the wash buffer, H₂O is substituted for the elution buffer, and elution is performed twice for 5 minutes each. The DNA is precipitated by adding 1/10 volume 3 M sodium acetate and 2.5 volumes 100% EtOH, incubated at -70°C for 30 minutes, centrifuged for 15 minutes, and washed with 70% EtOH.

The amine-modified DNA is resuspended in 5 µL H₂O. To the solution is added 3 µL 25 mg/mL sodium bicarbonate and 50 µg of Compound 9 (Reference dyes) or 69 (Reference dyes) in 5 µL DMF. The reaction is incubated for 1 hour at room temperature in the dark, to the reaction is added 90 µL H₂O, and it is purified using a QIAQUICK PCR purification kit (QIAGEN), with the following modifications: three washes are performed with 75% EtOH and three elutions of 5 minutes each with the Q**I**AGEN elution buffer. The DNA is precipitated as before. The labeled DNA products are suitable for *in situ* hybridization experiments, use on microarrays and as fluorescence donors or acceptors in hybridization-based assays. A comparison of varying the amino-allyl dUTP to dTTP ratios, followed by subsequent conjugation of the cDNA with either Compound 9 (Reference dyes) or Cy5 is shown in the Table 8. In the presence of excess reactive dye, both Compound 9 (Reference dyes) and Cy5 derivatives can label cDNA to equivalent degrees.

| **Table 8** | | |
|---|---|---|
| Nucleotide ratio, AA-dUTP:dTTP | Compound 9 | Cy5 |
| | bases/dye | bases/dye |
| 90µM:10µM | 13.2 | 13.6 |
| 60µM:10µM | 14.8 | 16.1 |
| 30µM:10µM | 17.6 | 15 |
| 10µM:10µM | 18.1 | 18.9 |
| 3µM:10µM | 23.3 | 23.7 |
| 1µM:10µM | 30.2 | 32.2 |

### Example 60. Comparison of the absorbance and fluorescence characteristics of nucleic acids.

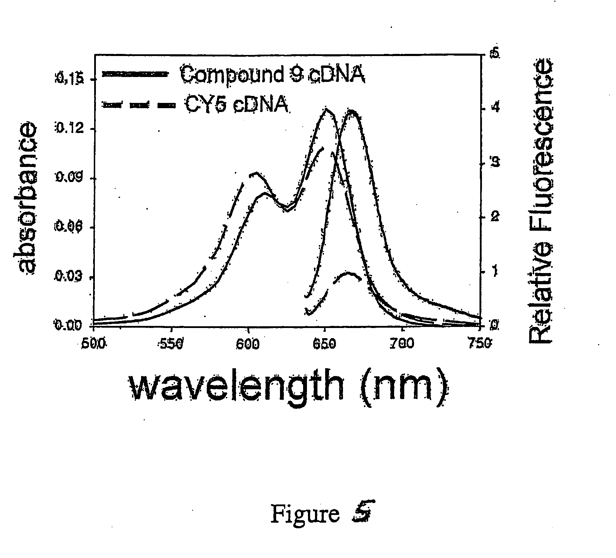

Nucleic acid conjugates of Compound 9 (Reference dye) and of Cy5 monosuccinimidyl ester are prepared from the same batch of amine-substituted nucleic acid (Example 59). Using the above protocol, it is possible to select a dye-labeling ratio that is essentially identical for both the Cy5 derivative and the Compound 9 derivative (e.g., 1 dye per 23 to 24 bases, Table 5). Absorption spectra at the same nucleic acid concentration and dye labeling ratio show a shifting of extinction from the long-wavelength 650 nm band to a non-emitting 600 nm band for the Cy5 conjugate relative to the conjugate of Compound 9 (Figure 5). This result is very similar to the absorbance changes observed for Cy5 conjugates of proteins. Fluorescence emission spectra excited at 600 nm reveal -4X greater fluorescence of the conjugate of Compound 9 cDNA relative to the Cy5 cDNA conjugate (Figure 5).

The synthesized Cy5 labeled cDNA is digested with micrococcal nuclease (EC 3.1.31.1; Worthington Biochemicals). The enzyme is dissolved at 1.0 mg/mL in reagent-grade water and diluted to approximately 0.001 mg/mL in 0.1% bovine serum albumin prior to addition to the Cy5 dye- and Compound 9-labeled cDNA's, pH = 8.8, 0.1 M sodium borate, 0.01 M calcium chloride. The reaction is allowed to proceed at room temperature for 4 hours.

A comparison of the absorbance spectra of the identical labeled cDNA before and after treatment with the nuclease (which will digest the cDNA to dNMP's) shows that the distortion in the absorbance of the Cy5-cDNA conjugate can be eliminated by digesting the cDNA down to its individual deoxynucleoside monophosphates. There is a concomitant increase in the fluorescence of the Cy5 cDNA upon digestion of ~15X, revealing that this absorbance pattern change is associated with the large decrease in the fluorescence associated with the Cy5-cDNA conjugate.

### Example 61. Discrimination of live and dead cells using the dyes of the invention.

Selected dyes of the invention are highly polar and therefore relatively impermeant to the membranes of live cells. These dyes can therefore be used to discriminate cells that have intact versus compromised cell membranes in a single-color assay as follows: Mouse monocyte-macrophage, Abelson Leukemia Virus Transformed (RAW264.7) cells are trypsinized and washed with PBS, pH 7.2. Approximately 8-10 million cells suspended in 180 µL of PBS, pH 7.2 are placed in a glass test tube and heated in a water bath at 50°C for 20 minutes to kill a fraction of the cells. Approximately 60 µL (2-3 million cells) of the cell suspension is added to 940 µL of PBS, pH 7.2, followed by 0.1 µL of a 1 mg/mL solution of a succinimidyl ester derivative of a dye of the invention m DMSO. The mixture is incubated on ice for 30 minutes and washed twice with PBS, followed by addition of 200 µL of PBS, pH 7.2. An identical aliquot of cells is treated with 2 µL of a 150 µM solution of propidium iodide in water (as a control for dead cells). Analysis of the cell suspension using flow cytometry shows that populations of dead cells stained by the instant Compounds and those stained by propidium iodide are very similar.

## Claims

1. A compound of the formula: 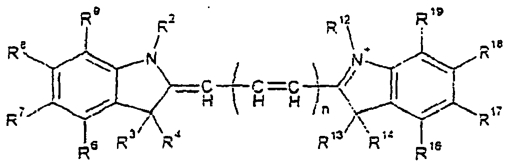 wherein:
n is 1, 2 or 3;
R² and R¹² are the same and are selected from methyl and ethyl ;
R³ is L-Rₓ or L-S_{c}; wherein
L is a single covalent bond, or a covalent linkage that is linear or branched, cyclic or heterocyclic, saturated or unsaturated, having 1-20 non hydrogen atoms selected from the group consisting of C, N, P, O, and S, such that the linkage contains any combination of ether, thioether, amine, ester, amide bonds; or single, double, triple or aromatic carbon-carbon bonds; or phosphorus-oxygen, phosphorus-suffur, nitrogen-nitrogen, nitrogen-oxygen, or nitrogen-platinum bonds; or aromatic or heteroaromatic bonds,
Rₓ is a carboxylic acid, an activated ester of a carboxylic acid, a reactive amine or a maieimide;
S_{c} is a conjugated substance;
R³, R¹³ and R¹⁴ are each independently C₁-C₆ alkyl optionally substituted one or more times by fluorine, chlorine, bromine, iodine, hydroxyl, carboxy, sulfo or amino;
R⁶, R⁸ and R⁹ are H and R⁷ is sulfo; and
R¹⁶, R¹⁸ and R¹⁹ are H and R¹⁷ is sulfo.

2. The compound of claim 1 wherein n is 1.

3. The compound of claim 1 wherein n is 2.

4. The compound of claim 1 wherein n is 3.

5. The compound of any preceding claim wherein L is a single covalent bond, or a covalent linkage that is linear or branched, cyclic or heterocyclic, saturated or unsaturated, having 1-16 non hydrogen atoms selected from the group consisting of C, N, P, O, and S, such that the linkage contains any combination of ether, thioether, amine, ester, amide bonds; or single, double, triple or aromatic carbon-carbon bonds; or phosphorus-oxygen, phosphorus-sulfur, nitrogen-nitrogen, nitrogen-oxygen, or nitrogen-platinum bonds; or aromatic or heteroaromatic bonds.

6. The compound of any preceding claim wherein L incorporates the formula -(CH₂)_{d}(CONH(CH₂)ₑ)_{z'} where d is 0-5, e is 1-5 and z' is 0 or 1.

7. The compound of claim 6 wherein L is -(CH₂)_{d}(CONH(CH₂)ₑ)_{z'} where d is 0-5, e is 1-5 and z' is 0 or 1.

8. The compound of any preceding claim wherein one of R¹³ and R¹⁴ is methyl and the other is C₁-C₆ alkyl substituted with carboxy, sulfo or hydroxy.

9. The compound of any preceding claim wherein R⁴ is a C₁-C₆ alkyl.

10. The compound of any preceding claim wherein R⁴, R¹³ and R¹² are methyl.

11. The compound of any preceding claim wherein R³ is L-Rₓ.

12. The compound of any preceding claim wherein Rₓ is a carboxylic acid, a succinimidyl ester of a carboxylic acid or a maleimide.

13. The compound of any of claims 1 to 10 wherein S_{c} is an antigen, steroid, vitamin, drug, hapten, metabolite, toxin, environmental pollutant, amino acid, peptide, protein, nucleic acid, nucleic acid polymer, carbohydrate, lipid, ion-complexing moiety, or glass, plastic or other non-biological polymer.

14. The compound of any of claims 1 to 10 wherein Sc is an amino acid, peptide, protein, tyramine, polysaccharide, ion-complexing moiety, nucleoside, nucleotide, oligonucleotide, nucleic acid, hapten, psoralen, drug, hormone, lipid, lipid assembly, polymer, polymeric microparticle, biological cell or virus.

15. The compound of any of claims 1 to 10 wherein Sc is a biological polymer such as a peptide, protein, oligonucleotide, or nucleic acid polymer that is also labelled with at least a second non-fluorescent or fluorescent dye (optionally an additional dye of the present invention), to form an energy-transfer pair.

16. A use of a compound of any of claims 1 to 15 as a dye to locate or detect the interaction or presence of analytes or ligands in a sample.

17. A method of staining a biological sample comprising: combining a dye solution comprising a compound of any of any of claims 1-15 with a biological sample in a concentration sufficient to yield a detectable optical response under the desired conditions.

18. A method of claim 17 wherein the sample comprises cells; proteins or nucleic acid polymers in a microarray; or a ligand that is a member of a specific binding pair, for which S_{c} is a complementary member.

## Patentansprüche

1. Verbindung der Formel: 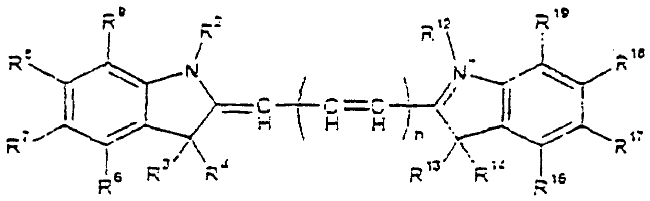 wobei:
n = 1, 2 oder 3 ist;
R² und R¹² gleich sind und ausgewählt werden aus Methyl und Ethyl;
R³ L-Rₓ oder L-S_{c} ist; wobei
L eine einzelne kovalente Bindung oder eine kovalente Verknüpfung, die linear oder verzweigt, cyclisch oder heterocyclisch, gesättigt oder ungesättigt ist und 1 bis 20 Nicht-Wasserstoffatome ausgewählt aus der Gruppe bestehend aus C, N, P, O und S aufweist, so, dass die Verknüpfung eine beliebige Kombination von Ether-, Thioether-, Amin-, Ester-, Amidbindungen; oder Einzel-, Doppel-, Dreifach- oder aromatische Kohlenstoff-Kohlenstoff-Bindungen; oder Phosphor-Sauerstoff-, Phosphor-Schwefel-, Stickstoff-Stickstoff-, Stickstoff-Sauerstoff- oder Stickstoff-Platin-Bindungen; oder aromatische oder heteroaromatische Bindungen enthält;
Rₓ eine Carbonsäure, ein aktivierter Ester einer Carbonsäure, ein reaktives Amin oder Maleimid ist;
S_{c} ein konjugierter Stoff ist;
R⁴, R¹³ und R¹⁴ jeweils unabhängig C₁-C₆-Alkyl, optional ein- oder mehrfach durch Fluor, Chlor, Brom, Jod, Hydroxy, Carboxy, Sulfo oder Amino substituiert, sind;
R⁶, R⁸ und R⁹ H ist und R⁷ Sulfo ist; und
R¹⁶, R¹⁸ und R¹⁹ H ist und R¹⁷ Sulfo ist.

2. Verbindung gemäß Anspruch 1, wobei n gleich 1 ist.

3. Verbindung gemäß Anspruch 1, wobei n gleich 2 ist.

4. Verbindung gemäß Anspruch 1, wobei n gleich 3 ist.

5. Verbindung gemäß einem vorhergehenden Anspruch, wobei L eine einzelne kovalente Bindung oder eine kovalente Verknüpfung, die linear oder verzweigt, cyclisch oder heterocyclisch, gesättigt oder ungesättigt ist und 1 bis 16 Nicht-Wasserstoffatome ausgewählt aus der Gruppe bestehend aus C, N, P, O und S aufweist, so, dass die Verknüpfung eine beliebige Kombination von Ether-, Thioether-, Amin-, Ester-, Amidbindungen; oder Einzel-, Doppel-, Tripel- oder aromatische Kohlenstoff-Kohlenstoff-Bindungen; oder Phosphor-Sauerstoff-, Phosphor-Schwefel-, Stickstoff-Stickstoff-, Stickstoff-Sauerstoff- oder Stickstoff-Platin-Bindungen; oder aromatische oder heteroaromatische Bindungen enthält.

6. Verbindung gemäß einem vorhergehenden Anspruch, wobei L die Formel-(CH₂)_{d}(CONH(CH₂)ₑ)_{z'} einschließt, wobei d 0 bis 5 ist, e 1 bis 5 ist und z' 0 oder 1 ist.

7. Verbindung gemäß Anspruch 6, wobei L -(CH₂)_{d}(CONH(CH₂)ₑ)_{z'} ist, wobei d 0 bis 5 ist, e 1 bis 5 ist und z' 0 oder 1 ist.

8. Verbindung gemäß einem vorhergehenden Anspruch, wobei eines von R¹³ und R¹⁴ Methyl ist und das andere C₁-C₆-Alkyl, substituiert mit Carboxy, Sulfo oder Hydroxy ist.

9. Verbindung gemäß einem vorhergehenden Anspruch, wobei R⁴ ein C₁-C₆-Alkyl ist.

10. Verbindung gemäß einem vorhergehenden Anspruch, wobei R⁴, R¹³ und R¹⁴ Methyl sind.

11. Verbindung gemäß einem vorhergehenden Anspruch, wobei R³ L-Rₓ ist.

12. Verbindung gemäß einem vorhergehenden Anspruch, wobei Rₓ eine Carbonsäure, ein Succinimidester einer Carbonsäure oder ein Maleimid ist.

13. Verbindung gemäß einem der Ansprüche 1 bis 10, wobei S_{c} ein Antigen, Steroid, Vitamin, Arzneimittel, Hapten, Metabolit, Toxin, umweltverunreinigender Stoff, Aminosäure, Peptid, Protein, Nukleinsäure, Nukleinsäurepolymer, Kohlenhydrat, Lipid, Ionenkomplexierendes Teil oder Glas, Plastik oder ein anderes nicht-biologisches Polymer ist.

14. Verbindung gemäß einem der Ansprüche 1 bis 10, wobei S_{c} eine Aminosäure, Peptid, Protein, Tyramin, Polysaccharid, Ion-komplexierendes Teil, Nukleosid, Nukleotid, Oligonukleotid, Nukleinsäure, Hapten, Psoralen, Arzneimittel, Hormon, Lipid, Lipidanordnung, Polymer, polymeres Mikropartikel, biologische Zelle oder Virus ist.

15. Verbindung gemäß einem der Ansprüche 1 bis 10, wobei S_{c} ein biologisches Polymer, wie ein Peptid, Protein, Oligonukleotid oder Nukleinsäurepolymer ist, das außerdem mit mindestens einem zweiten nichtfluoreszierenden oder fluoreszierenden Farbstoff (optional einem weiteren Farbstoff der vorliegenden Erfindung) markiert ist, um ein Energietransferpaar zu bilden.

16. Verwendung einer Verbindung gemäß einem der Ansprüche 1 bis 15 als Farbstoff, um die Interaktion oder Gegenwart von Analyten oder Liganden in einer Probe zu lokalisieren oder nachzuweisen.

17. Verfahren zum Färben einer biologischen Probe, das umfasst: das Kombinieren einer Farbstofflösung, umfassend eine Verbindung gemäß einem der Ansprüche 1 bis 15 mit einer biologischen Probe in einer Konzentration, die ausreicht, um ein nachweisbares optisches Ansprechen unter den gewünschten Bedingungen herbeizuführen.

18. Verfahren gemäß Anspruch 17, wobei die Probe Zellen; Proteine oder Nukleinsäurepolymere in einem Mikroarray; oder einen Liganden, der ein Mitglied eines spezifischen Bindepaars ist, für das S_{c} ein Komplementärmitglied ist, umfasst.

## Revendications

1. Composé de formule: 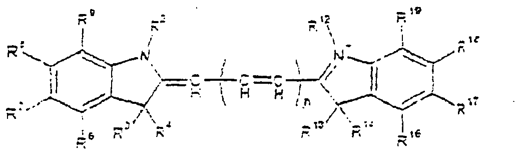 dans laquelle
n est 1, 2 ou 3;
R² et R¹² sont identiques et sont choisis parmi méthyle et éthyle;
R³ est L-Rₓ ou L-S_{c}; où
L est une liaison simple covalente, ou un enchaînement covalent qui est linéaire ou ramifié, cyclique ou hétérocyclique, saturé ou insaturé, ayant 1-20 atomes non hydrogène choisis dans le groupe constitué par C, N, P, O et S, de façon que l'enchaînement contienne une combinaison quelconque de liaisons éther, thioéther, amine, ester, amide; ou de liaisons carbone-carbone simples, doubles, triples ou aromatiques; ou de liaisons phosphore-oxygène, phosphore-soufre, azote-azote, azote-oxygène, ou azote-platine; ou de liaisons aromatiques ou hétéroaromatiques;
Rₓ est un acide carboxylique, un ester activé d'un acide carboxylique, une amine réactive ou un maléimide;
S_{c} est une substance conjuguée;
R⁴ R¹³ et R¹⁴ sont chacun indépendamment un alkyle en C₁-C₆ éventuellement substitué une ou plusieurs fois par du fluor, du chlore, du brome, de l'iode ou des groupes hydroxyle, carboxy, sulfo ou amino;
R⁶ R⁸ et R⁹ sont H et R⁷ est sulfo; et
R¹⁶, R¹⁸ et R¹⁹ sont H et R¹⁷ est sulfo.

2. Composé selon la revendication 1, dans lequel n est 1.

3. Composé selon la revendication 1, dans lequel n est 2.

4. Composé selon la revendication 1, dans lequel n est 3.

5. Composé selon l'une quelconque des revendications précédentes, dans lequel L est une liaison simple covalente, ou un enchaînement covalent qui est linéaire ou ramifié, cyclique ou hétérocyclique, saturé ou insaturé, ayant 1-16 atomes non hydrogène choisis dans le groupe constitué par C, N, P, O et S, de façon que l'enchaînement contienne une combinaison quelconque de liaisons éther, thioéther, amine, ester, amide; ou de liaisons carbone-carbone simples, doubles, triples ou aromatiques; ou de liaisons phosphore-oxygène, phosphore-soufre, azote-azote, azote-oxygène, ou azote-platine; ou de liaisons aromatiques ou hétéroaromatiques.

6. Composé selon l'une quelconque des revendications précédentes, dans lequel L incorpore la formule -(CH₂)_{d}(CONH(CH₂)ₑ)_{z'}, où d est 0-5, e est 1-5 et z' est 0 ou 1.

7. Composé selon la revendication 6, dans lequel L est -(CH₂)_{d}(CONH(CH₂)ₑ)_{z'}, où d est 0-5, e est 1-5 et z' est 0 ou 1.

8. Composé selon l'une quelconque des revendications précédentes, dans lequel l'un des R¹³ et R¹⁴ est un méthyle et l'autre est un alkyle en C₁-C₆ substitué par carboxy, sulfo ou hydroxy.

9. Composé selon l'une quelconque des revendications précédentes, dans lequel R⁴ est un alkyle en C₁-C₆.

10. Composé selon l'une quelconque des revendications précédentes, dans lequel R⁴, R¹³ et R¹⁴ sont des groupes méthyle.

11. Composé selon l'une quelconque des revendications précédentes, dans lequel R³ est L-Rₓ.

12. Composé selon l'une quelconque des revendications précédentes, dans lequel Rₓ est un acide carboxylique, un ester de succinimidyle d'un acide carboxylique, ou un maléimide.

13. Composé selon l'une quelconque des revendications 1 à 10, dans lequel S_{c} est un antigène, un stéroïde, une vitamine, un médicament, un haptène, un métabolite, une toxine, un polluant environnemental, un aminoacide, un peptide, une protéine, un acide nucléique, un polymère d'acides nucléiques, un hydrate de carbone, un lipide, un groupe complexant d'ions, ou un verre, une matière plastique ou un autre polymère non biologique.

14. Composé selon l'une quelconque des revendications 1 à 10, dans lequel S_{c} est un aminoacide, un peptide, une protéine, une tyramine, un polysaccharide, un groupe complexant d'ions, un nucléoside, un nucléotide, un oligonucléotide, un acide nucléique, un haptène, du psoralène, un médicament, une hormone, un lipide, un assemblage de lipides, un polymère, une microparticule de polymère, une cellule biologique ou un virus.

15. Composé selon l'une quelconque des revendications 1 à 10, dans lequel S_{c} est un polymère biologique comme un peptide, une protéine, un oligonucléotide ou un polymère d'acides nucléiques qui est aussi marqué avec au moins un deuxième colorant non fluorescent ou fluorescent (éventuellement un colorant de la présente invention supplémentaire), pour former une paire de transfert d'énergie.

16. Utilisation d'un composé selon l'une quelconque des revendications 1 à 15 comme colorant pour localiser ou détecter l'interaction ou la présence d'analytes ou de ligands dans un échantillon.

17. Procédé de coloration d'un échantillon biologique, comprenant la combinaison avec un échantillon biologique d'une solution de colorant comprenant un composé selon l'une quelconque des revendications 1-15 en une concentration suffisante pour donner une réponse optique détectable dans les conditions désirées.

18. Procédé selon la revendication 17, dans lequel l'échantillon comprend des cellules; des protéines ou des polymères d'acides nucléiques dans une biopuce; ou un ligand qui est un membre d'une paire de liaison spécifique pour laquelle S_{c} est un membre complémentaire.
